(19) Europäisches Patentamt / European Patent Office / Office européen des brevets

(11) **EP 4 186 520 A1**

(12) **EUROPEAN PATENT APPLICATION**
published in accordance with Art. 153(4) EPC

(43) Date of publication:
**31.05.2023 Bulletin 2023/22**

(21) Application number: **21847090.4**

(22) Date of filing: **19.07.2021**

(51) International Patent Classification (IPC):
*A61K 38/48* [(2006.01)]   *A61K 39/395* [(2006.01)]
*A61K 45/06* [(2006.01)]   *A61P 35/00* [(2006.01)]
*A61P 35/04* [(2006.01)]   *C07K 16/28* [(2006.01)]
*A61K 39/00* [(2006.01)]

(52) Cooperative Patent Classification (CPC):
**A61K 38/48; A61K 39/00; A61K 39/395;
A61K 45/06; A61P 35/00; A61P 35/04; C07K 16/28**

(86) International application number:
**PCT/KR2021/009225**

(87) International publication number:
**WO 2022/019584 (27.01.2022 Gazette 2022/04)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **23.07.2020 KR 20200091709
22.01.2021 KR 20210009360
09.06.2021 KR 20210074544**

(71) Applicant: **Medytox Inc.
Chungcheongbuk-do 28126 (KR)**

(72) Inventors:
• **KWAK, Seongsung**
  **Suwon-si Gyeonggi-do 16228 (KR)**
• **KIM, Taekyeong**
  **Suwon-si Gyeonggi-do 16263 (KR)**
• **LEE, Hyo Jin**
  **Seoul 06800 (KR)**
• **KANG, Won-ho**
  **Yongin-si Gyeonggi-do 16923 (KR)**
• **YANG, Gi Hyeok**
  **Seoul 06544 (KR)**

(74) Representative: **Brevalex
56, Boulevard de l'Embouchure
B.P. 27519
31075 Toulouse Cedex 2 (FR)**

(54) **CANCER THERAPEUTIC AGENT**

(57) A cancer therapeutic agent used in combination with an immunotherapeutic agent, for example, an immune checkpoint inhibitor, is disclosed.

EP 4 186 520 A1

# FIG. 1

MOUSE OF MALIGNANT MELANOMA (B16-F10)

Legend:
- VEHICLE
- BOTULINUM NEUROTOXIN
- ANTI-PD-1
- BOTULINUM NEUROTOXIN + ANTI-PD-1

Y-axis: TUMOR VOLUME (MEAN $mm^3 \pm$ SEM)

X-axis: NUMBER OF DAYS AFTER B16-F10 TUMOR TRANSPLANTATION

**Description**

**Technical Field**

The present disclosure relates to a cancer therapeutic agent, and more specifically, to a cancer therapeutic agent including botulinum neurotoxin as an active ingredient and used in combination with an immunotherapeutic agent.

**Background Art**

Botulinum neurotoxin (BoNT) is a polypeptide product of an anaerobic bacterium, Clostridium botulinum, and is a toxic substance specifically acting on nerve cells. Botulinum neurotoxin is a toxic substance that is inherently lethal, but has been used for treatment of cervical dystonia (CD), blepharospasm, hyperhidrosis, strabismus, achalasia, neurogenic bladder, urologic disease, migraine, and the like.

International Publication WO2010/062955 discloses a method of inhibiting growth or transfer of a neoplasm, wherein botulinum neurotoxin in a therapeutically effective amount is administered in combination with an anticancer drug or anticancer therapy to a non-neoplastic area near the neoplasm, but the botulinum neurotoxin in the therapeutically effective amount does not penetrate the neoplasm. In addition, International Publication WO2006/094539 discloses a method of sensitizing cancer treatment by cytotoxic therapy, such as chemotherapy or radiation therapy, and a composition for the method, the method comprising administering a pharmaceutical composition containing botulinum neurotoxin to cancer tissues or cells.

Anticancer immunotherapy is a treatment that activates an immune system of a human body to increase autoimmunity so that immune cells attack cancer cells. Immunotherapeutic agents can be largely classified into immune checkpoint inhibitors, immune cell therapy agents, therapeutic antibodies, anticancer vaccines, and oncolytic viruses. An immune checkpoint inhibitor is an agent that attacks cancer cells in a way that the activity of an immune checkpoint protein involved in the T cell inhibition is blocked to activate T cells, and representative examples thereof are antibodies recognizing CTLA-4, PD-1, and PD-L1. An immune cell therapy agent is an agent that strengthens the cellular immunity against cancer cells by collecting, strengthening, and modifying T cells of the body of a patient, and then, by injecting the resultant T cells back into the body, Examples thereof are an NK cell therapy agent, a T cell therapy agent, a CAR-T cell therapy agent, and the like. A therapeutic antibody is an agent that attacks cancer cells when a drug is dissociated upon binding of an antibody-drug conjugate to cancer cells, and a representative example thereof is an antibody-drug conjugate (ADC). An anticancer vaccine is to attack cancer cells in a way that tumor-specific antigens belonging to cancer cells or proteins or peptide molecules capable of improving the overall immune responses in the body are administered to a cancer patient to activate the immune system and accordingly to activate the immune functions of the body. The anticancer virus is a cancer-attacking virus that acts on direct apoptosis of cancer cells, and furthermore, the activation of immune cells is induced to enhance the anticancer capability.

Until now, attempts to treat cancer by using botulinum neurotoxin in combination with an immunotherapeutic agent have not be known.

**Disclosure**

**Technical Problem**

A first object of the present disclosure is to provide a composition for treating cancer, comprising botulinum neurotoxin as an active ingredient and used in combination with an immunotherapeutic agent.

A second object of the present disclosure is to provide a method of treating cancer by administering botulinum neurotoxin in a therapeutically effective amount in combination with an immunotherapeutic agent to a subject in need thereof.

A third object of the present disclosure is to provide botulinum neurotoxin for use as a cancer therapeutic agent used in combination with an immunotherapeutic agent.

A fourth object of the present disclosure is to provide use of botulinum neurotoxin as a cancer therapeutic agent used in combination with an immunotherapeutic agent.

**Technical Solution**

A first aspect of the present disclosure relates to a composition for treating cancer, comprising botulinum neurotoxin as an active ingredient and used in combination with an immunotherapeutic agent.

A second aspect of the present disclosure relates to a method of treating cancer by administering botulinum neurotoxin in a therapeutically effective amount in combination with an immunotherapeutic agent to a subject in need thereof.

A third aspect of the present disclosure relates to botulinum neurotoxin for use as a cancer therapeutic agent used in combination with an immunotherapeutic agent.

A fourth aspect of the present disclosure relates to use of botulinum neurotoxin as a cancer therapeutic agent used in combination with an immunotherapeutic agent.

As used in the present specification, the term 'botulinum neurotoxin' refers to protein molecules of botulinum toxin including all serotypes and variants thereof or fusion proteins thereof and produced by bacteria or recombination.

A Clostridium botulinum strain produces immunologically distinct neurotoxins (NTXs, types A to G). NTX types A to G have molecular masses of about 150 kDa (7S). In culture media and foods having acidic conditions, an NTX binds to a non-toxic component to form a large complex called a progenitor toxin (PTX). PTXs having molecular masses of 900 kDa (19S), 500 kDa (16S), and 300 kDa (12S) have been discovered. The 12S toxin consists of an NTX and a non-toxic component having no hemagglutinin activity (designated as non-toxic non-hemagglutinin (NTNH)), and the 19S and 16S toxins each consist of an NTX, NTNH, and hemagglutinin. A type A strain produces toxins in three forms (19S, 16S, and 12S). Types B, C, and D strains produce 16S and 12S toxins. Under alkaline conditions, the PTX dissociates into an NTX and a non-toxic component, the 19S and 16S toxins each dissociate into an NTX, NTNH, and a non-toxic component (complex) of hemagglutinin, and the 12S toxin dissociates into an NTX and NTNH. In the present specification, the botulinum neurotoxin may be in any of the aforementioned forms, and for example, may have a molecular mass of the 19S, 16S, 12S, or 7S toxin.

The botulinum neurotoxin may include a botulinum neurotoxin derivative. The botulinum neurotoxin derivative may be a derivative having one or more chemical or functional modifications on a part or some chains of natural botulinum neurotoxin or recombinant native botulinum neurotoxin having botulinum neurotoxic activity. For example, the botulinum neurotoxin derivative may be a modified toxin having one or more amino acids deleted, modified, or substituted. The botulinum neurotoxin may be a recombinant peptide, a fusion protein, or for example, a hybrid neurotoxin prepared from subunits or domains of different toxin serotypes. The botulinum neurotoxin may also be a part of an entire molecule having toxic activity, and a combination thereof or a part of conjugated molecules, such as fusion proteins, may be used.

In one aspect, the botulinum neurotoxin may be botulinum neurotoxin that does not have any chemical or functional modification. An example thereof is natural botulinum neurotoxin or recombinant native botulinum neurotoxin, or a part thereof, such as complexed botulinum neurotoxin (e.g., 19S toxin) or non-complexed botulinum neurotoxin (e.g., 7S toxin).

The term 'immunotherapeutic agent' refers to a compound, composition, or treatment that indirectly or directly enhances, stimulates, or increases immune responses of the body against cancer cells and/or that reduces side effects of other anticancer therapies. Non-limiting examples of the immunotherapeutic agents are cytokines, cancer vaccines, monoclonal antibodies, non-cytokine adjuvants, anticancer viruses, immune cells (T cells, NK cells, dendritic cells, B cells, etc.), immune checkpoint inhibitors, and the like.

The expression 'used in combination' refers to any form of two or more different therapeutic agents used in a way that a second therapeutic agent is administered while a previously administered therapeutic agent is still effective in the body. For example, such two types of therapeutic agents may be simultaneously effective in a subject, expecting a synergistic effect of the two types of therapeutic agents. Such different therapeutic agents may be administered simultaneously or sequentially in the form of a single formulation or separate formulations.

The term 'administration' or 'administering' refers to a step of providing a pharmaceutical composition or an active ingredient to a subject. A pharmaceutical composition may be administered via a variety of suitable routes.

The term 'pharmaceutical composition' refers to a formulation containing an active ingredient. The term 'formulation' refers that, in addition to an active ingredient (e.g., botulinum neurotoxin), at least one additional ingredient, such as albumin (human serum albumin or recombinant human albumin) and/or sodium chloride, is present in the pharmaceutical composition. The pharmaceutical composition is a formulation suitable for administration to a subject such as a human patient. The pharmaceutical composition may be in a lyophilized form, for example, a solution formed after reconstitution of a lyophilized pharmaceutical composition using saline or water, or may be in the form of a solution that does not require reconstitution. The pharmaceutical composition may be in a liquid state or a solid state. The pharmaceutical composition may be free of animal-derived proteins and/or albumin.

The term 'therapeutically effective amount' refers to a level, amount, or concentration of an agent, such as botulinum neurotoxin or a pharmaceutical composition including botulinum neurotoxin, required to treat a disease, disorder, or abnormality without causing any significantly negative or adverse side effect.

The term 'treat', 'treating', or 'treatment' refers to, for example, healing of injured or damaged tissue, or alleviation or reduction (including partial, substantial, near complete, or complete reduction), remedy or prevention (either temporary or permanent) of a disease, disorder, or abnormality to achieve a desired therapeutic outcome by altering, changing, enhancing, ameliorating, improving, and/or beatifying an existing or recognized disease, disorder, or abnormality. In the present specification, the term 'treatment' refers to a concept including prevention. The term 'prevention' refers to delaying the onset of a disease, disorder, or illness. When the onset of a disease, disorder, illness is delayed for a pre-determined period of time, prevention may be considered complete.

In one aspect, the term 'treatment' refers to treatment of a disease, disorder, or medical condition in a patient, such as a mammal (particularly a human), including one or more of the following:

(a) prevention of a disease, disorder, or medical condition from occurrence, i.e. prevention of a disease or medical condition from recurrence, or prophylactic treatment of a patient pre-disposed to the disease or medical condition;

(b) amelioration of a disease, disorder, or medical condition, i.e., elimination or regression of a disease, disorder, or medical condition in a patient, including an antagonism against effects of other therapeutic agents;

(c) suppression of a disease, disorder, or medical condition, i.e., slowdown or blocking of the development of a disease, disorder, or medical condition in a patient; or

(d) alleviation of symptoms of a disease, disorder, or medical condition in a patient.

The present disclosure is based on the discovery that botulinum neurotoxin or a composition including the same can be used in combination with an immunotherapeutic agent to be utilized as a therapeutic agent for cancer.

The present disclosure is based on the discovery that botulinum neurotoxin or a composition including the same can be used in combination with an immunotherapeutic agent to be utilized as a therapeutic agent for metastatic cancer.

In one aspect, the botulinum neurotoxin may be used without limitation to serotypes, and may be selected from the group consisting of types A (BoNT/A), B (BoNT/B), C (BoNT/C), D (BoNT/D), and E (BoNT/E ), F (BoNT/F), G (BoNT/G), H (BoNT/H), X (BoNT/X), Enterococcus spp. botulinum neurotoxin J (eBoNT/J), and mosaic botulinum neurotoxin, and/or variants thereof. Examples of the mosaic botulinum neurotoxin are BoNT/DC, BoNT/CD, and BoNT/FA. For example, BoNT/A may be used.

In one aspect, the botulinum neurotoxin may have various BoNT/A subtypes, such as A1, A2, A3, A4, A5, A6, A7, A9, A10; BoNT/B subtypes, such as B1, B2, B3, B4, B5, B6, B7, B8, Bnp, and Bbv; BoNT/C subtypes, such as C and CD; BoNT/D subtypes, such as D and DC; BoNT/E subtypes, such as E1, E2, E3, E4, E5, E6, E7, E8, and E9; BoNT/F subtypes, such as F1, F2, F3, F4, F5, F6, and F7; and BoNT/G subtype, such as G. The BoNT subtypes include chimeric BoNT, for example BoNT/DC, BoNT/CD, BoNT/FA, and the like.

In one aspect, the immunotherapeutic agent may use any material or therapy known in the art, and may include cytokines, cancer vaccines, oncolytic viruses, monoclonal antibodies, non-cytokine adjuvants, immune cells (T cells, NK cells, dendritic cells, B cells, etc.), and immune checkpoint inhibitors. In an embodiment, the immunotherapeutic agent may be an immune checkpoint inhibitor. The immune checkpoint inhibitor may include a peptide, an antibody, a nucleic acid molecule, and a small molecule. For example, the immune checkpoint inhibitor may be administered to enhance the proliferative, migratory, and persistent properties and/or cytotoxic activity of CD8+T cells in a subject, and in particular, to enhance the tumor invasiveness of CD8+T cells in a subject. Typically, the immune checkpoint inhibitor may be an antagonist that blocks activated T lymphocytes, such as cytotoxic T lymphocyte-associated protein 4 (CTLA4) and programmed cell death 1 (PD-1), or immunosuppressive receptors expressed by NK cells, such as various members of the killer cell immunoglobulin-like receptor (KIR) family, or may be an antagonist that blocks key ligands of these receptors, such as PD-1 ligand CD274 (best known as PD-L1 or B7-H1). For example, the immune checkpoint inhibitor may be an antibody or an antigen-binding fragment thereof. In an embodiment, the immune checkpoint inhibitor may be one or more selected from the group consisting of an anti-PD-1 antibody, an anti-PD-L1 antibody, an anti-PD-L2 antibody, an anti-CTLA-4 antibody, an anti-TIM-3 antibody, an anti-LAG3 antibody, an anti-IDO1 antibody, an anti-TIGIT antibody, an anti-B7H3 antibody, an anti-B7H4 antibody, an anti-BTLA antibody, and an anti-B7H6 antibody, and an antigen-binding fragment thereof. In one or more embodiments, the immune checkpoint inhibitor may be one or more selected from the group consisting of ipilimumab (Yervoy®, BMS/Ono), tremelimumab (AstraZeneca), atezolizumab (Tecentriq®, Roche), nivolumab (Opdivo®, BMS/Ono), pembrolizumab (Keytruda®, MSD), avelumab (Bavencio®, Pfizer/Merck,

Germany), durvalumab (Imfinzi®, AstraZeneca/MedImmune), and an antigen-binding fragment thereof, but is not limited thereto.

In one aspect, the cancer may be exosome-mediated.

Exosomes are a type of extracellular vesicles and have a diameter of approximately 100 nm. These are enclosed by a lipid bilayer and are secreted from most cells. The exosomes are produced as intraluminal membrane vesicles (ILVs) in the endocytic system and are secreted during fusion of cell membranes with multivesicular bodies (MVBs). The exosomes have been reported to contribute to the maintenance of tissue homeostasis by regulating cell-cell communication. Exosomes released from cancer cells are involved in cancer progression. Proliferative cancer cells (1) expand cancer tissue by using angiogenesis, (2) acquire the ability to migrate and invade, and (3) acquire the ability to evade attack from immune cells, and ultimately form metastatic lesions, and exosomes may be involved in each of these processes.

In one aspect, the cancer may be a solid tumor.

Non-limiting examples of the solid cancer are breast cancer, lung cancer, head or neck cancer, colorectal cancer, esophageal cancer, laryngeal cancer, stomach cancer, liver cancer, pancreatic cancer, bone cancer, skin cancer, skin or intraocular melanoma, uterine cancer, ovarian cancer, rectal cancer, anal cancer, colon cancer, breast cancer, fallopian tube carcinoma, endometrial carcinoma, cervical carcinoma, vaginal carcinoma, vulvar carcinoma, Hodgkin's disease, small intestine cancer, endocrine cancer, thyroid cancer, parathyroid cancer, adrenal cancer, soft tissue sarcoma, urethral cancer, penile cancer, prostate cancer, chronic or acute leukemia, lymphocytic lymphoma, bladder cancer, kidney or ureter cancer, renal cell carcinoma, renal pelvic carcinoma, CNS tumor, primary CNS lymphoma, spinal cord tumor, and brain tumor such as glioma (e.g. astrocytoma, glioblastoma, oligodendroglioma, and ependymoma, blastocytoma, meningioma, brainstem glioma, pituitary adenoma, schwannoma, congenital tumor, craniopharyngioma, and metastatic brain tumor). For example, the solid tumor may include one or more selected from the group consisting of melanoma, colorectal cancer, breast cancer, lung cancer, pancreatic cancer, prostate cancer, bladder cancer, and stomach cancer, but is not limited thereto.

In one aspect, the cancer may be metastatic cancer.

In one aspect, the composition may be used in a subject with increased secretion of the exosomes.

In one aspect, the composition including botulinum neurotoxin may further include one or more pharmaceutically acceptable excipients or additives.

The pharmaceutically acceptable excipient or additive may include a stabilizer, an ionic compound, a surfactant, a buffer, a lyoprotectant, or a combination thereof, and for example, may include an amino acid (e.g., methionine), a salt (e.g., NaCl), a buffer solution, a non-ionic surfactant (e.g., polysorbate such as polysorbate 20), sugar (e.g., a disaccharide such as sucrose or the like), sugar alcohol (e.g., sorbitol), or a combination thereof.

The composition may not include albumin or an animal-derived component or polysaccharide.

The composition may include, for example, those disclosed in WO 2009-008595 A1 or WO2012-134240 A2, and the descriptions of these patents are hereby incorporated by reference in their entirety.

For use as the composition, a commercially available pharmaceutical composition including botulinum neurotoxin may be used without limitation. For example, BOTOX® (botulinum neurotoxin type A neurotoxin complex of human serum albumin and sodium chloride) (Allergan, Inc.: Irvine, California, USA), DYSPORT® (powder formulation that is reconstituted with 0.9% sodium chloride prior to use; clostridium botulinum type A toxin hemagglutinin complex of human serum albumin and lactose in the formulation) (Ipsen Limited, Berkshire, UK), MYOBLOC™ (an injectable solution containing botulinum neurotoxin type B, human serum albumin, sodium succinate, and sodium chloride, approximately pH 56) (Solstice Neurosciences, Inc.: South San Francisco, California, USA), Meditoxin® (lyophilized powder formulation containing clostridium botulinum neurotoxin type A, human serum albumin, and sodium chloride) (Medytox Inc., Korea), Innotox® (liquid formulation containing clostridium botulinum neurotoxin type A, L-methionine, polysorbate 20, sodium chloride, and water for injection) (Medytox Inc., Korea), or Coretox® (lyophilized powder formulation containing clostridium botulinum neurotoxin type A (150 kD), polysorbate 20, sucrose, and sodium chloride) (Medytox Inc., Korea) may be used. In an embodiment, the composition may be Meditoxin®, Innotox®, Coretox®, or a combination of one or more thereof, but is not limited thereto.

In one aspect, the composition may be formulated in any form, e.g., solid or liquid formulation, such as lyophilized powder, liquid, or pre-filled syringe formulation.

In one aspect, the composition may be formulated for local administration. For example, such local administration may include direct administration to a tumor or a precancerous tissue or to a surrounding area of a tumor or a precancerous tissue, and typically, may be achieved by intratumoral injection or peritumoral injection of an aqueous solution of botulinum neurotoxin. For example, the formulation may be administered under the skin or into muscles, such as soft tissue. By intratumoral or peritumoral injection at a certain location, the composition can inhibit the tumor growth or block the oncogenesis at a location away from the certain location, and thus can exhibit a systemic effect by local administration.

In one aspect, a therapeutically effective amount of botulinum neurotoxin may be about 0.01 U/kg to about 100 U/kg, about 0.1 U/kg to about 100 U/kg, about 0.2 U/kg to about 100 U/kg, about 0.2 U/kg to about 50 U/kg, about 0.2 U/kg to about 30 U/kg, about 0.2 U/kg to about 10 U/kg, or about 0.2 U/kg to about 1 U/kg. In one or more aspects, based on an adult weighing 60 kg, the therapeutically effective amount may be about 1 U to about 10,000 U, from about 1 U to about 5,000 U, from about 1 U to about 2,500 U, from about 1 U to about 1,000 U, from about 1 U to about 500 U, about 1 U to about 300 U, about 1 U to about 200 U, about 10 U to about 200 U, about 10 U to about 100 U, about 10 U to about 50 U.

As used in the present specification, the term "unit", "unit(s)", or "U" refers to a LD50 dose defined as the amount of botulinum neurotoxin that kills 50 % of mice injected with botulinum neurotoxin, and may be used interchangeably within one product.

In one aspect, botulinum neurotoxin may be administered in a single session or multiple treatment sessions.

The administration may be performed at a dose in the form of a single injection or split injections at an injection site. In multiple treatment sessions, botulinum neurotoxin may be administered at intervals of 6 months or less. In multiple treatment sessions, the administration interval of botulinum neurotoxin includes primary treatment and secondary treatment, and a dosage of the secondary treatment may be less than, greater than, or the same as the dosage of the primary treatment.

Doses, administration time, administration methods, and administration periods or intervals of botulinum neurotoxin may be appropriately selected and used by those skilled in the art according to weight, age, gender, health condition, severity of disease, and the like of a patient.

In one aspect, botulinum neurotoxin may be administered simultaneously or sequentially with the immunotherapeutic agent.

In one aspect, botulinum neurotoxin may be administered in a single formulation or in separate formulations with the immunotherapeutic agent.

In one aspect, the immunotherapeutic agent may be formulated by a conventional method according to each purpose of use in various forms, such as oral formulations including solutions, suspensions, powders, granules, tablets, capsules, pills, extracts, emulsions, syrups, aerosols, and the like, and parenteral formulations including injections of sterile injection solution. The immunotherapeutic agent may be administered orally or parenterally administered via a variety of routes. For example, the immunotherapeutic agent may be locally administered or injected intravenously, intraperitoneally, subcutaneously, intradermally, or intramuscularly, or into the spine, spinal cavity, or rectum. The doses may be in vary ranges depending on weight, age, gender, health condition, diet, administration time, administration method, administration period or interval, excretion rate, and constitutional specificity of a patient, nature of a formulation, severity of a disease, and the like, and may be appropriately selected by those skilled in the art. For example, the dose may be in a range of about 0.1 mg/kg to about 10,000 mg/kg, but is not limited thereto, and the administration may be performed once a day or splitly several times a day.

In one aspect, a method of treating exosome-mediated cancer may include the following steps:

1) quantifying exosomes secreted from a biological sample obtained from a subject; and

2) administering a therapeutically effective amount of botulinum neurotoxin in combination with an immunotherapeutic agent as a single formulation or separate formulations to a subject having increased secretion of exosomes, either simultaneously or sequentially.

The secreted exosomes may be isolated and quantified from a biological sample of a subject, for example, blood, according to a conventional method known in the art, and there are no particular limitations on a sample type and a method for separation and quantification.

The subject to which botulinum neurotoxin or the composition including the same is administered may include humans or animals without limitation, such as humans, pigs, dogs, cats, cows, horses, and mice.

## Advantageous Effects

According to the present disclosure, botulinum neurotoxin or a composition including the same may be used in combination with an immunotherapeutic agent, such as an immune checkpoint inhibitor, to be useful as a cancer therapeutic agent.

## Description of Drawings

FIG. 1 is a graph showing the tumor growth over time after botulinum neurotoxin is administered in combination with an anti-PD-1 antibody to a mouse model transplanted with malignant melanoma described in Example 1.

FIG. 2A is a graph showing the ratios of CD4+T cells based on total viable cells infiltrated in a tumor after botulinum neurotoxin is administered in combination with an anti-PD-1 antibody to a mouse model transplanted with malignant melanoma described in Example 1.

FIG. 2B is a graph showing the ratios of CD8+T cells based on total viable cells infiltrated in a tumor after botulinum neurotoxin is administered in combination with an anti-PD-1 antibody to a mouse model transplanted with malignant melanoma described in Example 1.

FIG. 2C is a graph showing the number of CD4+T cells in an actual tumor based on total viable cells infiltrated in a tumor after botulinum neurotoxin is administered in combination with an anti-PD-1 antibody to a mouse model transplanted with malignant melanoma described in Example 1.

FIG. 2D is a graph showing the number of CD8+T cells in an actual tumor based on total viable cells infiltrated in a tumor after botulinum neurotoxin is administered in combination with an anti-PD-1 antibody to a mouse model transplanted with malignant melanoma described in Example 1.

FIG. 3 is a graph showing the number of exosomes in blood of a vehicle group and each experimental group after botulinum neurotoxin is administered in combination with an anti-PD-1 antibody to a mouse model transplanted with malignant melanoma described in Example 1.

FIG. 4 is a graph showing the tumor growth over time after botulinum neurotoxin is administered in combination with an anti-PD-1 antibody by dose to a mouse model transplanted with malignant melanoma described in Example 2.

FIG. 5 is a graph showing the survival rate of a mouse after botulinum neurotoxin is administered in combination with an anti-PD-1 antibody by dose to a mouse model transplanted with malignant melanoma described in Example 2.

FIG. 6 is a graph showing the tumor growth over time after an anti-PD-1 antibody is administered with complex botulinum neurotoxin or non-complex botulinum neurotoxin to a mouse model transplanted with malignant melanoma described in Example 3.

FIG. 7 is a graph showing the tumor growth over time after botulinum neurotoxin is administered in combination with an anti-CTLA4 antibody to a mouse model transplanted with pulmonary tumor described in Example 4.

FIG. 8A is a graph showing the tumor growth on the right over time after botulinum neurotoxin is administered in combination with an anti-PD-1 antibody to a mouse model transplanted with malignant melanoma on both sides as described in Example 5.

FIG. 8B is a graph showing the tumor growth on the left over time after botulinum neurotoxin is administered in combination with an anti-PD-1 antibody to a mouse model transplanted with malignant melanoma on both sides as described in Example 5.

FIG. 9 is a graph showing the survival rate of a mouse after botulinum neurotoxin is administered in combination with an anti-PD-1 antibody to a mouse model transplanted with malignant melanoma on both sides as described in Example 5.

FIG. 10A is a graph showing the ratio of CD11 b+cells based on viable cells infiltrated on the left side of non-administered tumors in a mouse model transplanted with malignant melanoma on both sides as described in Example 5.

FIG. 10B is a graph showing the ratio of CD4+T cells based on viable cells infiltrated on the left side of non-administered tumors in a mouse model transplanted with malignant melanoma on both sides as described in Example 5.

FIG. 10C is a graph showing the ratio of CD8a+T cells based on viable cells infiltrated on the left side of non-administered tumors in a mouse model transplanted with malignant melanoma on both sides as described in Example 5.

FIG. 11 is a graph showing the tumor growth over time after botulinum neurotoxin is administered in combination with an anti-PD-1 antibody or propranolol is administered in combination with an anti-PD-1 antibody to a mouse model transplanted with colorectal cancer as described in Example 6.

FIG. 12 is a graph showing the survival rate of a mouse after botulinum neurotoxin is administered in combination with an anti-PD-1 antibody or propranolol is administered in combination with an anti-PD-1 antibody to a mouse model transplanted with colorectal cancer as described in Example 6.

FIG. 13 is a graph showing the tumor growth over time after botulinum neurotoxin is administered in combination with an anti-CTLA4 antibody to a mouse model transplanted with colorectal cancer as described in Example 6.

FIG. 14 is a graph showing the survival rate of a mouse after botulinum neurotoxin is administered in combination with an anti-CTLA4 antibody to a mouse model transplanted with colorectal cancer as described in Example 6.

**Best Mode**

Hereinafter, the present disclosure will be described in more detail with Examples, but these are only for explaining the present disclosure and are not intended to limit the scope in any way.

**Example 1: Anticancer efficacy test of botulinum neurotoxin and anti-PD-1 antibody on mouse model of malignant melanoma transplantation**

6-week-old male C57BL/6 mice were purchased (Orient Bio), and after acclimation and quarantine for 1 week, 7-week-old mice were used in experiments. For a feed, a sterilized solid feed for laboratory animals (R40-10, SAFE, France) was freely fed, and for drinking water, tap water was autoclaved and freely fed. During the periods of acclimatization, quarantine, and experiments, the mice were bred under specific-pathogen-free conditions set at a temperature of 23 $\pm$ 3 °C, relative humidity of 55 $\pm$ 15 %, lighting time of 12 hours (from 8:00 am to 8:00 pm), ventilation frequency of 15 times/hour, and illumination of 150 Lux to 300 Lux. This experiment was performed after review and approval (A-2020-004) by Institutional Animal Care and Use Committee of Medytox Inc.

A B16-F10 mouse malignant melanoma cell line (KCLB No: 80008) was obtained from the Korean Cell Line Bank (KCLB). B16-F10 cells were cultured in a DMEM medium (CAT No: 11965-092, Gibco) supplemented with 10 % FBS (CAT No: 10082-147, Gibco) and 1% penicillin-streptomycin (CAT No: 15140-122, Gibco) in an incubator at 37 °C and 5 % $CO_2$ conditions. 0.1 ml of $5 \times 10^5$ cells were transplanted into the right flank of anesthetized C57BL/6 mice by using a syringe.

Sterile physiological saline (Lot No: M8T7AF3, Daehan Pharmaceutical Co., Ltd.) used for preparing botulinum neurotoxin was used as a vehicle, and Coretox 100 unit (Lot No: NSA19007A, Medytox) was used as botulinum neurotoxin. Anti-PD-1 antibodies (Clone: RMP1-14, Cat No: BE0146, Lot No: 760220J1) were purchased from Bio X cell for use.

Each test material was prepared and administered according to Table 1 below. 6.667 ml of sterile physiological saline was added to a vial containing 100 units of botulinum neurotoxin to be prepared at a concentration of 15 units/ml, and then intratumorally administered at a dose of 1 ml/kg or 15 units/kg. An anti-PD-1 antibody was prepared at a final concentration of 1 mg/ml by using sterile PBS (Cat No: 10010, Gibco) on the day of administration, and then administered intraperitoneally at a dose of 5 ml/kg or 5 mg/kg.

On the 8th day after the mice were transplanted with malignant melanoma, the body weight and tumor size of the mice were measured, and when having the size of about 33 mm$^3$ in average, 10 mice were randomly assigned per test group to have no difference among the test groups. On the 8th day after the tumor transplantation, a vehicle and botulinum neurotoxin were intratumorally administered according to Table 1 based on the body weight. An anti-PD-1 antibody was intraperitoneally administered on the 8th day, 11th day, 14th day, and 17th day after the tumor transplantation according to Table 1. The tumor size was measured twice a week after the tumor transplantation. The tumor size was measured with a vernier caliper, and the tumor volume was calculated by using the following equation with the measured values.

$$\text{Tumor volume (mm}^3) = (W2xL)/2$$

W (mm)=width (short axis of tumor), L (mm)=length of tumor (long axis of tumor)

$$\text{Tumor growth inhibition (TGI, \%)} = (1 - \text{average tumor size in test group/average tumor size in vehicle}) \times 100$$

[Table 1]

| Group | Number of animal | Material to be administered | Dose | Administration route | Administration frequency |
|---|---|---|---|---|---|
| 1 | 9* | Vehicle | 1 ml/kg, 5 ml/kg | Intratumoral, intraperitoneal | Once (on 8th day after tumor transplantation), twice a week, 4 times in total |
| 2 | 10 | Botulinum neurotoxin | 15 units/kg | Intratumoral | Once (on 8th day after tumor transplantation) |
| 3 | 10 | Anti-PD-1 antibody | 5 mg/kg | Intraperitoneal | Twice a week, 4 times in total |
| 4 | 9* | Botulinum neurotoxin+anti-PD-1 antibody | 15 units/kg, 5 mg/kg | Intratumoral, intraperitoneal | Once (on 8th day after tumor transplantation), twice a week, 4 times in total |

[0063]   * From each of Test Groups 1 and 4, one mouse was excluded due to a severe wound around the tumor caused by a fight between individuals.

On day 19th day after the tumor transplantation, all animals were euthanized before the tumor size reached 2,000 mm$^3$. After completion of the euthanasia, the tumor tissue was excised and weighed, and the number of T cells in the tumor tissue was measured by using a flow cytometer (FACSVerse, BD). For the analysis of T cells in the tumor tissue, the tumor tissue was separated into single cells by using gentle MACS C tubes (Cat No: 130-093-237, Miltenyi Biotec), and then stained with a LIVE/DEAD Fixable Aqua Dead Cell Stain (Cat No: L34957, Invitrogen), an Fc block (Cat No: 553142, BD), and CD45-PECy7 (Cat No: 25-0451-82, eBioscience), TCRbeta-FITC (Cat No: 109206, Biolegend), CD4-pacific blue (Cat No: 100428, Biolegend ), CD8a-PerCPCy55 (Cat No: 100734, Biolegend) antibodies to be analyzed by flow cytometry. The number of CD4+ and CD8+ T cells in the tumor was calculated based on the tumor weight, and the ratio was represented as a ratio of CD45+TCRbeta+CD4+ and CD45+TCRbeta+CD8a+ T cells based on viable cells.

Exosomes in blood were isolated by using an Exoquick exosome precipitation solution (Cat No: EXOQ20A-1, Lot No: 200107-001, System Biosciences), and then measured by using an ExoELISA-ULTRA Complete Kit (Cat No: EXEL-ULTRA-CD63-1, Lot No: 200226-002, System Biosciences).

A Graphpad Prism (version 7.05, GraphPad Software Inc., CA, USA) was used for the graph presentation, and an SPSS software (version 25.0, SPSS Inc., IL, USA) was used for the statistical analysis. A two-tailed t-test was performed with respect to parametric data, and Mann-Whitney test statistical analysis was performed with respect to non-parametric data at a significance level of p=0.05.

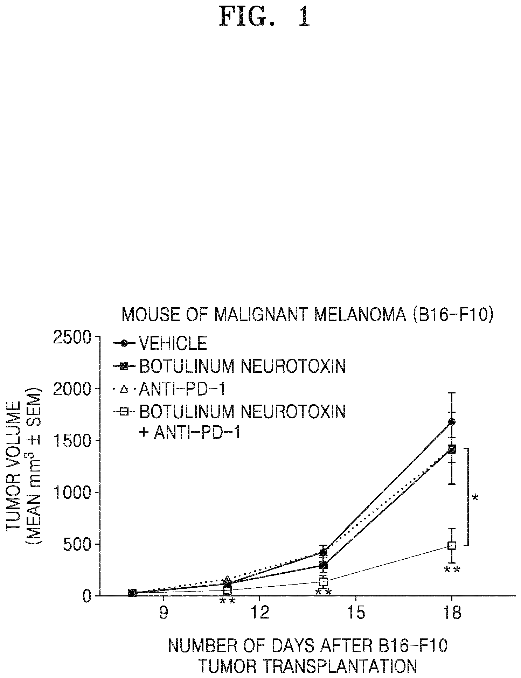

The results of measuring the tumor growth inhibition are shown in Table 2 and FIG. 1.

[Table 2]

| Group | Material to be administered | Average tumor size[1] (mean mm$^3$±SEM) | Average tumor weight[2] (mean mm$^3$±SEM) | TGI (%)[1] |
|---|---|---|---|---|
| 1 | Vehicle | 1684±284 | 2.02±038 | 0 |
| 2 | Botulinum neurotoxin | 1433±348 | 1.29±035 | 15 |
| 3 | Anti-PD-1 antibody | 1415±121 | 1.83±018 | 16 |
| 4 | Botulinum neurotoxin+anti-PD-1 antibody | 489±165**, †, ‡ | 0.46±0.12**, †, ‡ | 71 |
| | 1) Results at the 18th day after tumor transplantation, 2) results at the 19th day after tumor transplantation, **p<0.01, vs vehicle. †p<0.05, vs botulinum neurotoxin, ‡p<0.001, vs anti-PD-1 antibody, two-tailed t-test. | | | |

As shown in Table 2 and FIG. 1, on the 18th day after the mice were transplanted with malignant melanoma, the intratumoral administration of botulinum neurotoxin only showed an average tumor size of 1433 mm$^3$ and a tumor growth inhibition rate of 15 %, whereas the intratumoral administration of anti-PD-1 antibody only showed an average of 1415 mm$^3$ and a tumor growth inhibition rate of 16 %. However, there was no statistical significance compared to the vehicle-administered group. In contrast, when botulinum neurotoxin was administered in combination with anti-PD-1 antibody, an average tumor size was 489 mm$^3$, and a tumor growth inhibition rate was 71 %, confirming that the tumor growth was inhibited more significantly compared to the case where each of botulinum neurotoxin and an anti-PD-1 antibody was administered alone. In addition, even when the tumor was excised and weighed on the 19th day after the tumor transplantation, the case where botulinum neurotoxin was administered in combination with anti-PD-1 antibody showed a significant reduction in the tumor weight compared to the groups administered with vehicle only, botulinum neurotoxin only, and anti-PD-1 antibody only, respectively.

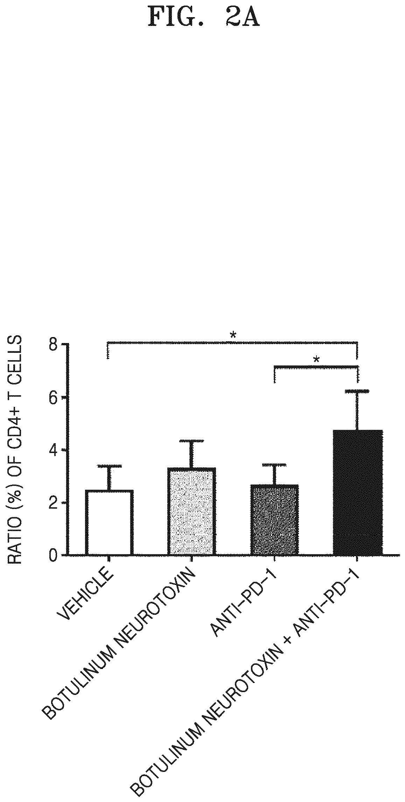

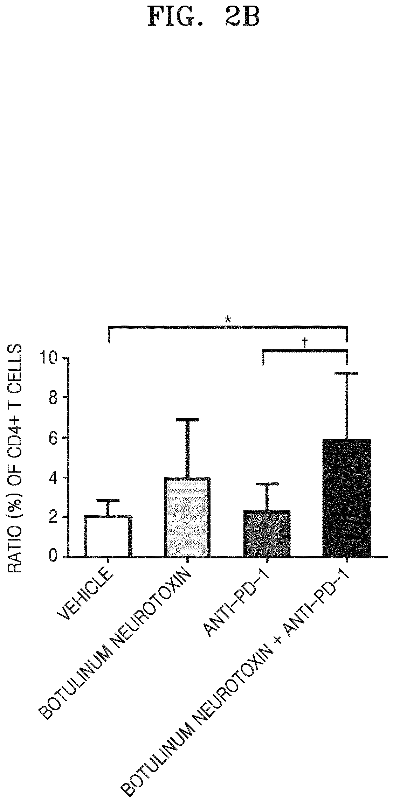

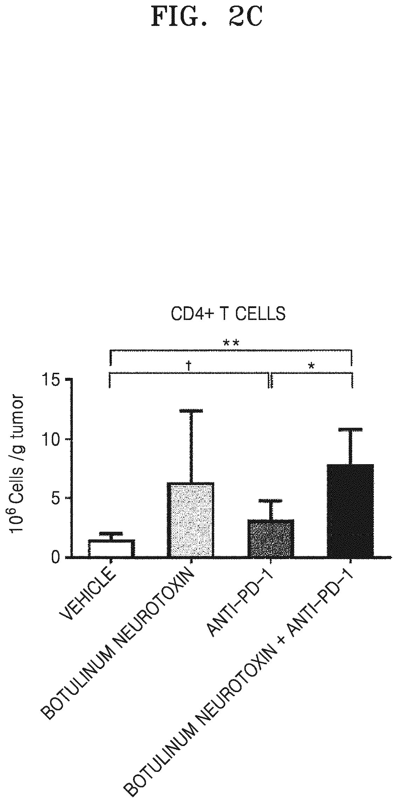

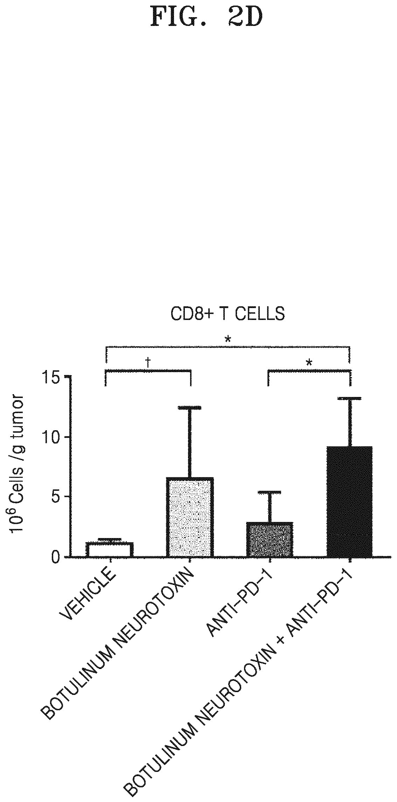

As a result of analyzing CD4+ and CD8+ T cells infiltrated into the tumor after the tumor tissue was excised, the case where only botulinum neurotoxin was administered showed a tendency to increase the number of CD8+ T cells in the tumor compared to the vehicle-treated group (FIGS. 2A and 2B). For the ratios of CD4+ and CD8+ T cells, there was no significant difference between the groups each administered with botulinum neurotoxin only and anti-PD-1 antibody only and the vehicle-treated group. However, in the group treated with botulinum neurotoxin in combination with anti-PD-1 antibody, a significantly high ratio of CD4+ and CD8+ T cells was confirmed to be infiltrated into the tumor compared to the vehicle-treated group (FIGS. 2C and 2D). That is, there was a significant increase compared to the group treated with anti-PD-1 antibody only. It can be explained that, in the group treated with botulinum neurotoxin and anti-PD-1 antibody together, T cells were significantly increased in the tumor due to a synergistic action between the two substances so that the tumor growth was significantly reduced.

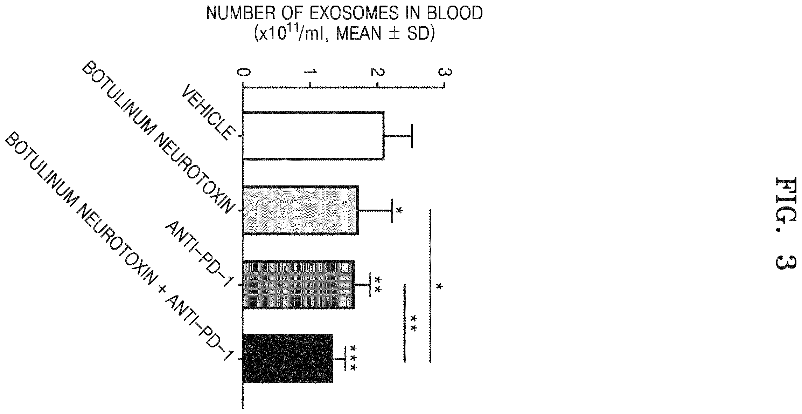

The results of measuring the number of exosomes in blood are shown in FIG. 3. As shown in FIG. 3, the number of exosomes in the blood tended to decrease in the group administered with botulinum neurotoxin only compared to the vehicle-treated group, and was significantly decreased in the group administered with anti-PD-1 antibody only and the group administered with botulinum neurotoxin+anti-PD-1 antibody. In addition, the group administered with botulinum neurotoxin in combination with anti-PD-1 antibody showed a significant decrease in the number of exosomes in the blood compared to the groups each administered with botulinum neurotoxin only and anti-PD-1 only, respectively.

**Example 2: Anticancer efficacy test of botulinum neurotoxin and anti-PD-1 antibody by dose on mouse model of malignant melanoma transplantation**

6-week-old male C57BL/6 mice were purchased (Orient Bio), and after acclimation and quarantine for 1 week, 7-week-old mice were used in experiments. For a feed, a sterilized solid feed for laboratory animals (R40-10, SAFE, France) was freely fed, and for drinking water, tap water was autoclaved and freely fed. During the periods of acclimatization, quarantine, and experiments, the mice were bred under specific-pathogen-free conditions set at a temperature of 23 ± 3 °C, relative humidity of 55 ± 15 %, lighting time of 12 hours (from 8:00 am to 8:00 pm), ventilation frequency of 15 times/hour, and illumination of 150 Lux to 300 Lux. This experiment was performed after review and approval (A-2020-004) by Institutional Animal Care and Use Committee of Medytox Inc.

A B16-F10 mouse malignant melanoma cell line (KCLB No: 80008) was obtained from the Korean Cell Line Bank (KCLB). B16-F10 cells were cultured in a DMEM medium (CAT No: 11965-092, Gibco) supplemented with 10 % FBS (CAT No: 10082-147, Gibco) and 1% penicillin-streptomycin (CAT No: 15140-122, Gibco) in an incubator at 37 °C and 5 % $CO_2$ conditions. 0.1 ml of $5 \times 10^5$ cells were transplanted into the right flank of anesthetized C57BL/6 mice by using a syringe.

A placebo of a botulinum neurotoxin product containing the same excipient components as in the botulinum neurotoxin product, but only botulinum neurotoxin was excluded was used as a vehicle, and Coretox 100 unit (Lot No: NSA19007A, Medytox) was used as botulinum neurotoxin. An anti-PD-1 antibody (Clone: RMP1-14, Cat No: BE0146, Lot No: 780120J2) was purchased from Bio X cell for use. Each test material was prepared and administered according to Table 3 below. Sterile physiological saline was added to a vial containing 100 units of botulinum neurotoxin to be prepared at concentrations of 1 unit/ml, 3 units/ml, and 10 units/ml, and then intratumorally administered at a dose of 1 ml/kg or 1 unit/kg, 3 units/kg, or 10 units/kg. An anti-PD-1 antibody was prepared at a final concentration of 1 mg/ml by using sterile PBS (Cat No: 10010, Gibco) on the day of administration, and then administered intraperitoneally at a dose of 5 ml/kg or 5 mg/kg.

On the 8th day after the mice were transplanted with malignant melanoma, the tumor size was measured, and 12 mice were randomly assigned per test group to have no difference among the test groups. On the 8th day after the tumor transplantation, a vehicle and botulinum neurotoxin were intratumorally administered according to the compositions of the test groups. In the case of anti-PD-1 antibody, intraperitoneal administration thereof was performed twice a week for a total of 4 times according to Table 3. The tumor size was measured twice a week after the tumor transplantation. The tumor size was measured with a vernier caliper, and the tumor volume was calculated by using the equation described in Example 1.

[Table 3]

| Group | Number of animal | Material to be administered | Dose | Administration route | Administration frequency |
|---|---|---|---|---|---|
| 1 | 12 | Vehicle | 1 ml/kg | Intratumoral | Once (on 8th day after tumor transplantation) |
| 2 | 12 | 1 U/kg botulinum neurotoxin | 1 ml/kg | Intratumoral | Once (on 8th day after tumor transplantation) |
| 3 | 12 | 3 U/kg botulinum neurotoxin | 1 ml/kg | Intratumoral | Once (on 8th day after tumor transplantation) |
| 4 | 12 | 10 U/kg botulinum neurotoxin | 1 ml/kg | Intratumoral | Once (on 8th day after tumor transplantation) |
| 5 | 12 | 1 U/kg botulinum neurotoxin+5 mg/kg anti-PD-1 antibody | 1 ml/kg, 5 ml/kg | Intratumoral, intraperitoneal | Once (on 8th day after tumor transplantation), twice a week, 4 times in total |
| 6 | 12 | 3 U/kg botulinum neurotoxin+5 mg/kg anti-PD-1 antibody | 1 ml/kg, 5 ml/kg | Intratumoral, intraperitoneal | Once (on 8th day after tumor transplantation), twice a week, 4 times in total |
| 7 | 12 | 10 U/kg botulinum neurotoxin+5 mg/kg anti-PD-1 antibody | 1 ml/kg, 5 ml/kg | Intratumoral, intraperitoneal | Once (on 8th day after tumor transplantation), twice a week, 4 times in total |
| 8 | 12 | Vehicle+5 mg/kg anti-PD-1 antibody | 1 ml/kg, 5 ml/kg | Intratumoral, intraperitoneal | Once (on 8th day after tumor transplantation), twice a week, 4 times in total |

After the tumor transplantation, measurements were performed until the tumor size in each mouse reached 2,000 mm$^3$, and subjects having the tumor size exceeding the euthanasia standard (2,000 mm$^3$) were immediately euthanized and recorded as dead.

A Graphpad Prism (version 7.05, GraphPad Software Inc., CA, USA) was used for the graph presentation, and an SPSS software (version 25.0, SPSS Inc., IL, USA) and an Excel (2013, MS, USA) were used for the statistical analysis. A two-tailed t-test was performed with respect to the tumor size, and Mantel-Cox log-rank analysis was performed with respect to the survival rates at a significance level of $p=0.05$.

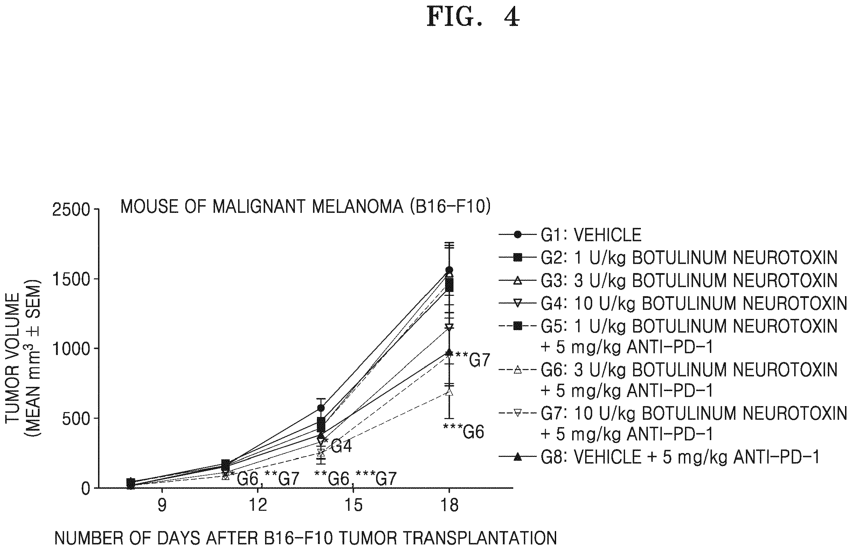

Results of the tumor size measurement are shown in FIG. 4 (*p<0.05, **p<0.01, and ***p<0.001). As shown in FIG. 4 , when botulinum neurotoxin was intratumorally administered in a single dose of 1 U/kg, 3 U/kg, or 10 U/kg to the mouse model of malignant melanoma, significant tumor growth inhibition was observed on the 14th day in in the 10 U/kg-administered group. On the 18th day after the tumor transplantation, no significant inhibitory effect on the tumor growth was observed in the test group administered with botulinum neurotoxin only and the test group administered with vehicle + 5 mg/kg of anti-PD1 antibody, compared to the vehicle-administered group. However, a significant inhibitory effect on the tumor growth was observed in the test group administered with 3 U/kg or 10 U/kg of botulinum neurotoxin in combination with 5 mg/kg of anti-PD1 antibody, compared to the vehicle-administered group.

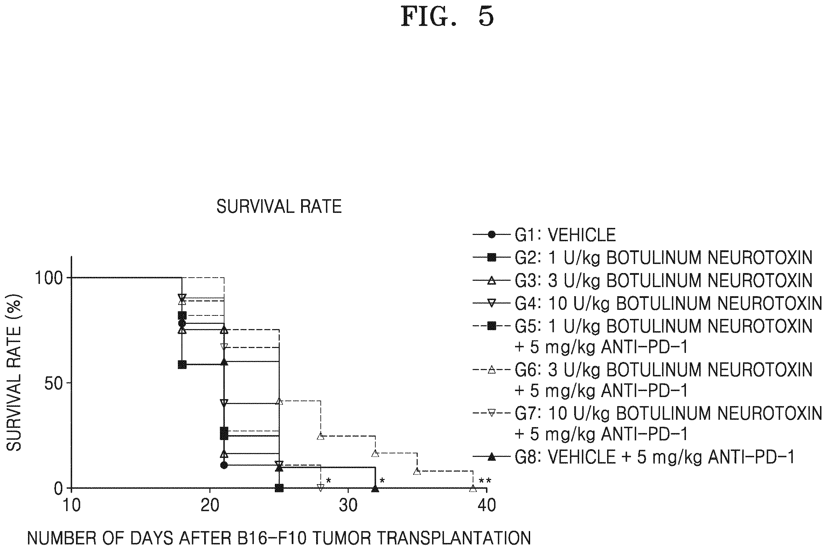

Results of the survival rate measurement are shown in FIG. 5. As shown in FIG. 5, the mouse survival rate was significantly increased in the test group administered with 3 U/kg or 10 U/kg of botulinum neurotoxin in combination with 5 mg/kg of anti-PD1 antibody. In addition, a significant increase in the survival rate was observed in the group administered with vehicle + 5 mg/kg of anti-PD1. Based on the results above, it was confirmed that there is an inhibitory effect on tumor growth in the mouse model of melanoma when botulinum neurotoxin was administered at a dose of 3 U/kg or more in combination with anti-PD1 antibody.

**Example 3: Anti-cancer efficacy test of complexed botulinum neurotoxin and non-complexed botulinum neurotoxin administered in combination with anti-PD-1 antibody on mouse model of malignant melanoma transplantation**

6-week-old male C57BL/6 mice were purchased (Orient Bio), and after acclimation and quarantine for 1 week, 7-week-old mice were used in experiments. For a feed, a sterilized solid feed for laboratory animals (R40-10, SAFE, France) was freely fed, and for drinking water, tap water was autoclaved and freely fed. During the periods of acclimatization, quarantine, and experiments, the mice were bred under specific-pathogen-free conditions set at a temperature of 23 ± 3 °C, relative humidity of 55 ± 15 %, lighting time of 12 hours (from 8:00 am to 8:00 pm), ventilation frequency of 15 times/hour, and illumination of 150 Lux to 300 Lux. This experiment was performed after review and approval (A-2020-004) by Institutional Animal Care and Use Committee of Medytox Inc.

A B16-F10 mouse malignant melanoma cell line (KCLB No: 80008) was obtained from the Korean Cell Line Bank (KCLB). B16-F10 cells were cultured in a DMEM medium (CAT No: 11965-092, Gibco) supplemented with 10 % FBS (CAT No: 10082-147, Gibco) and 1% penicillin-streptomycin (CAT No: 15140-122, Gibco) in an incubator at 37 °C and 5 % $CO_2$ conditions. 0.1 ml of $5 \times 10^5$ cells were transplanted into the right flank of anesthetized C57BL/6 mice by using a syringe.

A placebo of a non-complexed botulinum neurotoxin product containing the same excipient components as in the botulinum neurotoxin product, but only botulinum neurotoxin was excluded was used as a vehicle. Coretox 100 unit (Lot No: NSA19007A, Medytox) was used as non-complexed botulinum neurotoxin, Meditoxin 100 unit (Lot No: TFAA20024, Medytox) was used as complexed botulinum neurotoxin. An anti-PD-1 antibody (Clone: RMP1-14, Cat No: BE0146, Lot No: 780120J2) was purchased from Bio X cell for use. Each test material was prepared and administered according to Table 4 below. Sterile physiological saline was added to a vial containing 100 units of botulinum neurotoxin to be prepared at a concentration of 15 units/ml, and then intratumorally administered at a dose of 1 ml/kg or 15 units/kg. An anti-PD-1 antibody was prepared at a final concentration of 1 mg/ml by using sterile PBS (Cat No: 10010, Gibco) on the day of administration, and then administered intraperitoneally at a dose of 5 ml/kg or 5 mg/kg.

On the 8th day after the mice were transplanted with malignant melanoma, the tumor size was measured, and 12 mice were randomly assigned per test group to have no difference among the test groups. On the 8th day after the tumor transplantation, a vehicle and botulinum neurotoxin were intratumorally administered according to the compositions of the test groups. In the case of anti-PD-1 antibody, intraperitoneal administration thereof was performed twice a week for a total of 4 times according to Table 4. The tumor size was measured twice a week after the tumor transplantation. The tumor size was measured with a vernier caliper, and the tumor volume was calculated by using the equation described in Example 1.

[Table 4]

| Group | Number of animal | Material to be administered | Dose | Administration route | Administration frequency |
|---|---|---|---|---|---|
| 1 | 12 | Vehicle | 1 ml/kg | Intratumoral | Once (on 8th day after tumor transplantation) |
| 2 | 12 | 15 U/kg non-complexed botulinum neurotoxin | 1 ml/kg | Intratumoral | Once (on 8th day after tumor transplantation) |
| 3 | 12 | 15 U/kg complexed botulinum neurotoxin | 1 ml/kg | Intratumoral | Once (on 8th day after tumor transplantation) |
| 4 | 12 | 15 U/kg non-complexed botulinum | 1 ml/kg | Intratumoral | Once (on 8th day after tumor transplantation) |
|  |  | neurotoxin+5 mg/kg anti-PD-1 antibody |  |  |  |
| 5 | 12 | 15 U/kg complexed botulinum neurotoxin+5 mg/kg anti-PD-1 antibody | 1 ml/kg, 5 ml/kg | Intratumoral, intraperitoneal | Once (on 8th day after tumor transplantation), twice a week, 4 times in total |
| 6 | 12 | Vehicle+5 mg/kg anti-PD-1 antibody | 1 ml/kg, 5 ml/kg | Intratumoral, intraperitoneal | Once (on 8th day after tumor transplantation), twice a week, 4 times in total |

On day 19th day after the tumor transplantation, all animals were euthanized before the tumor size reached 2,000 $mm^3$, and the experiment was terminated.

A Graphpad Prism (version 7.05, GraphPad Software Inc., CA, USA) was used for the graph presentation, and an SPSS software (version 25.0, SPSS Inc, IL, USA) and an Excel (2013, MS, USA) were used for the statistical analysis. A two-tailed t-test was performed with respect to the tumor size at a significance level of p=0.05.

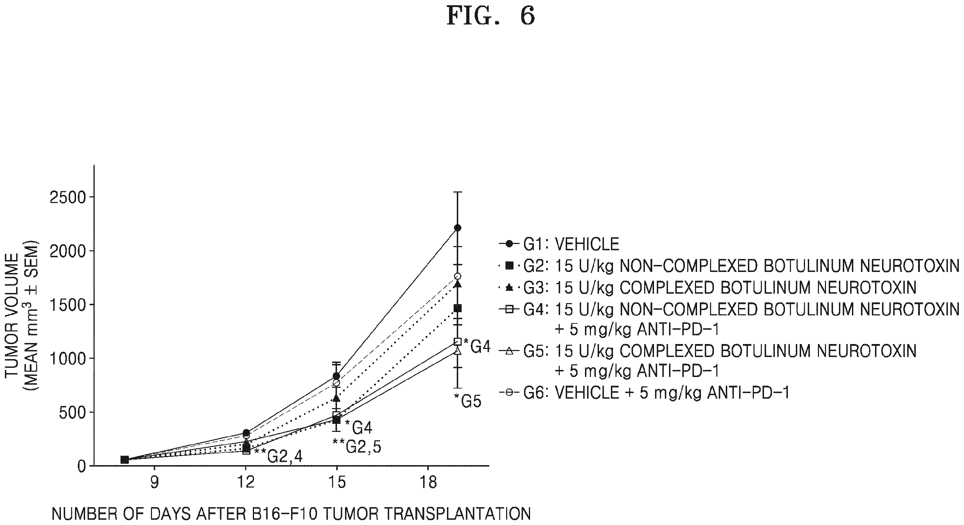

The results are shown in FIG. 6 (*$p < 0.05$, **$p < 0.01$). As shown in FIG. 6, on the 19th day after the mice were transplanted with malignant melanoma, significant inhibition of the tumor growth was observed in the case where 15 U/kg of non-complexed botulinum neurotoxin and 15 U/kg of complexed botulinum neurotoxin were each administered in combination with 5 mg/kg of anti-PD1 antibody. Here, no significant difference was observed among the test groups administered with the non-complexed botulinum neurotoxin only and the test groups administered with complexed botulinum neurotoxin only. In addition, no significant difference was observed among the groups administered with non-complexed botulinum neurotoxin+anti-PD1 antibody and the groups administered with complexed botulinum neurotoxin+anti-PD1 antibody. Based on the results above, it was confirmed that, in both a case where non-complexed botulinum neurotoxin was administered in combination with anti-PD1 antibody and a case where complexed botulinum neurotoxin was administered in combination with anti-PD1 antibody, an inhibitory effect on tumor growth was exhibited regardless of the type of botulinum neurotoxin.

**Example 4: Anticancer efficacy test of botulinum neurotoxin and anti-CTLA4 antibody on mouse model of pulmonary tumor transplantation**

6-week-old male C57BL/6 mice were purchased (Orient Bio), and after acclimation and quarantine for 1 week, 7-week-old mice were used in experiments. For a feed, a sterilized solid feed for laboratory animals (R40-10, SAFE, France) was freely fed, and for drinking water, tap water was autoclaved and freely fed. During the periods of acclimatization, quarantine, and experiments, the mice were bred under specific-pathogen-free conditions set at a temperature of $23 \pm 3$ °C, relative humidity of $55 \pm 15$ %, lighting time of 12 hours (from 8:00 am to 8:00 pm), ventilation frequency of 15 times/hour, and illumination of 150 Lux to 300 Lux. This experiment was performed after review and approval (A-2020-004) by Institutional Animal Care and Use Committee of Medytox Inc.

An LLC1 mouse lung cancer cell line (ATCC No: CRL-1642) was obtained from ATCC. LLC1 cells were cultured in a DMEM medium (CAT No: LM001-05, Welgene) supplemented with 10 % FBS (CAT No: 10082-147, Gibco) and 1 % penicillin-streptomycin (CAT No: 15140-122, Gibco) in an incubator at 37 °C and 5 % $CO_2$ conditions. 0.1 ml of $5 \times 10^5$ cells were transplanted into the right flank of anesthetized C57BL/6 mice by using a syringe.

A placebo of a botulinum neurotoxin product containing the same excipient components as in the botulinum neurotoxin product, but only botulinum neurotoxin was excluded was used as a vehicle, and Coretox 100 unit (Lot No: NSA19007A, Medytox) was used as botulinum neurotoxin. Anti-CTLA4 antibody (Clone: 9H10, Cat No: BE0131, Lot No: 704019A2) was purchased from Bio X cell for use. Each test material was prepared and administered according to Table 5 below. Sterile physiological saline was added to a vial containing 100 units of botulinum neurotoxin to be prepared at a concentration of 15 units/ml, and then intratumorally administered at a dose of 1 ml/kg or 15 units/kg. Anti-CTLA4 antibody was prepared at a final concentration of 1 mg/ml by using sterile PBS (Cat No: 10010, Gibco) on the day of administration, and then administered intraperitoneally at a dose of 5 ml/kg or 5 mg/kg.

On the 8th day after the mice were transplanted with LLC1 pulmonary tumor cell line, the tumor size was measured, and 10 mice were randomly assigned per test group to have no difference among the test groups. On the 8th day after the tumor transplantation, a vehicle and botulinum neurotoxin were intratumorally administered according to the compositions of the test groups. In the case of anti-CTLA4 antibody, intraperitoneal administration thereof was performed twice a week for a total of 4 times according to Table 5. The tumor size was measured twice a week after the tumor transplantation. The tumor size was measured with a vernier caliper, and the tumor volume was calculated by using the equation described in Example 1.

[Table 5]

| Group | Number of animal | Material to be administered | Dose | Administration route | Administration frequency |
|---|---|---|---|---|---|
| 1 | 10 | Vehicle | 1 ml/kg | Intratumoral | Once (on 8th day after tumor transplantation) |
| 2 | 10 | 15 U/kg botulinum neurotoxin | 1 ml/kg | Intratumoral | Once (on 8th day after tumor transplantation) |
| 3 | 10 | 15 U/kg botulinum neurotoxin+5 mg/kg anti-CTLA4 antibody | 1 ml/kg, 5 ml/kg | Intratumoral, intraperitoneal | Once (on 8th day after tumor transplantation), twice a week, 4 times in total |
| 4 | 10 | Vehicle+5 mg/kg anti-CTLA4 antibody | 1 ml/kg, 5 ml/kg | Intratumoral, intraperitoneal | Once (on 8th day after tumor transplantation), twice a week, 4 times in total |

On day 21st day after the tumor transplantation, all animals were euthanized before the tumor size reached

2,000 mm$^3$, and the experiment was terminated.

A Graphpad Prism (version 7.05, GraphPad Software Inc., CA, USA) was used for the graph presentation, and an SPSS software (version 25.0, SPSS Inc, IL, USA) and an Excel (2013, MS, USA) were used for the statistical analysis. A two-tailed t-test was performed with respect to the tumor size at a significance level of p=0.05.

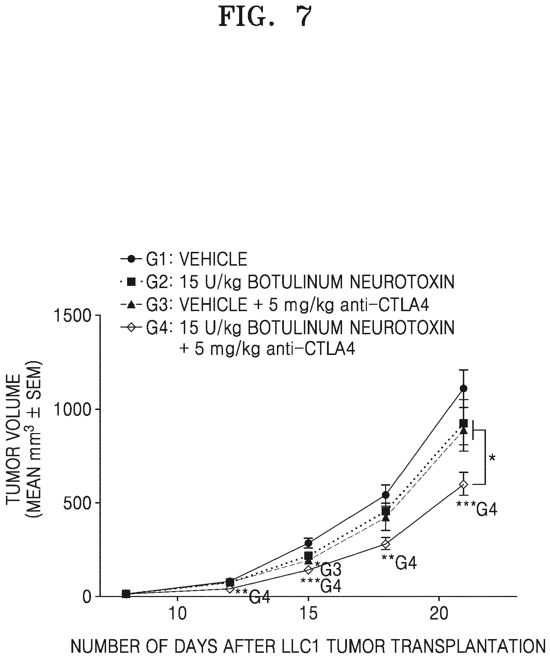

The results are shown in FIG. 7 (*p<0.05, **p<0.01, and ***$p$<0.001). As shown in FIG. 7, on the 21st day after the mice were transplanted with pulmonary tumor, no significant inhibitory effect on tumor growth was observed in the group administered with 15 U/kg of botulinum neurotoxin only or with vehicle+5 mg/kg of anti-CTLA4 antibody compared to the vehicle-treated test group, whereas a significant inhibitory effect on tumor growth was observed in the group administered with 15 U/kg of botulinum neurotoxin+5 mg/kg of anti-CTLA4 compared to the vehicle-treated test group. In addition, the tumor volume was significantly reduced in the group administered with 15 U/kg of botulinum neurotoxin+5 mg/kg of anti-CTLA4 compared to the test group administered with 15 U/kg of botulinum neurotoxin only and the group administered with vehicle+5 mg/kg of anti-CTLA4 antibody. Based on the results above, it was confirmed that the administration of botulinum neurotoxin in combination with anti-CTLA4 antibody showed an inhibitory effect on tumor growth in the mouse model of LLC1 pulmonary tumor.

**Example 5: Anticancer efficacy test of botulinum neurotoxin and anti-PD-1 antibody on mouse model of bilateral malignant melanoma transplantation**

6-week-old male C57BL/6 mice were purchased (Orient Bio), and after acclimation and quarantine for 1 week, 7-week-old mice were used in experiments. For a feed, a sterilized solid feed for laboratory animals (R40-10, SAFE, France) was freely fed, and for drinking water, tap water was autoclaved and freely fed. During the periods of acclimatization, quarantine, and experiments, the mice were bred under specific-pathogen-free conditions set at a temperature of 23 ± 3 °C, relative humidity of 55 ± 15 %, lighting time of 12 hours (from 8:00 am to 8:00 pm), ventilation frequency of 15 times/hour, and illumination of 150 Lux to 300 Lux. This experiment was performed after review and approval (A-2020-004) by Institutional Animal Care and Use Committee of Medytox Inc.

A B16-F10 mouse malignant melanoma cell line (KCLB No: 80008) was obtained from the Korean Cell Line Bank (KCLB). B16-F10 cells were cultured in a DMEM medium (CAT No: 11965-092, Gibco) supplemented with 10 % FBS (CAT No: 10082-147, Gibco) and 1 % penicillin-streptomycin (CAT No: 15140-122, Gibco) in an incubator at 37 °C and 5 % $CO_2$ conditions. 0.1 mL of $5\times10^5$ cells were transplanted into the right flank of the anesthetized C57BL/6 mice by using a syringe, and after 5 days, 0.1 mL of $2\times10^5$ cells to $2.5\times10^5$ cells were transplanted into the left flank of the anesthetized C57BL/6 mice by using a syringe.

A placebo of a botulinum neurotoxin product containing the same excipient components as in the botulinum neurotoxin product, but only botulinum neurotoxin was excluded was used as a vehicle, and Coretox 100 unit (Lot No: NSA20015, Medytox) was used as botulinum neurotoxin. An anti-PD-1 antibody (Clone: RMP1-14, Cat No: BE0146, Lot No: 780120J2 or 798921J1C) was purchased from Bio X cell for use. Each test material was prepared and administered according to Table 6 below. Sterile physiological saline was added to a vial containing 100 units of botulinum neurotoxin to be prepared at a concentration of 15 units/mL, and then intratumorally administered at a dose of 1 mL/kg or 15 units/kg. An anti-PD-1 antibody was intraperitoneally administered at a dose of 5 mg/kg with PBS (Cat No: LB004-02, Welgene).

The experiment was repeated twice with the same test groups according to the same procedures. On the 8th day after the mice were transplanted with malignant melanoma on the right flank, the tumor size was measured, and 12 mice were randomly assigned per test group to have no difference among the test groups. On day 8 after the tumor transplantation on the right flank, a vehicle and botulinum neurotoxin were intratumorally administered on the right flank according to the compositions of the test groups. In the case of anti-PD-1 antibody, intraperitoneal administration thereof was performed twice a week for a total of 4 times according to the compositions of the test groups. After the tumor transplantation, the size of the tumors transplanted on the right and left sides was measured twice a week. The tumor size was measured with a vernier caliper, and the tumor volume was calculated by using the equation described in Example 1.

[Table 6]

| Group | Number of animal | Material to be administered | Dose | Administration route | Administration frequency |
|---|---|---|---|---|---|
| 1 | 12 | Vehicle | 1 ml/kg | Intratumoral | Once (on 8th day after tumor transplantation) |
| 2 | 12 | 15 U/kg botulinum neurotoxin | 1 ml/kg | Intratumoral | Once (on 8th day after tumor transplantation) |

(continued)

| Group | Number of animal | Material to be administered | Dose | Administration route | Administration frequency |
|---|---|---|---|---|---|
| 3 | 12 | Vehicle+5 mg/kg anti-PD-1 antibody | 1 ml/kg, 5 ml/kg | Intratumoral, intraperitoneal | Once (on 8th day after tumor transplantation), twice a week, 4 times in total |
| 4 | 12 | 15 U/kg botulinum neurotoxin+5 mg/kg anti-PD-1 antibody | 1 ml/kg, 5 ml/kg | Intratumoral, intraperitoneal | Once (on 8th day after tumor transplantation), twice a week, 4 times in total |

On the 21st day after the tumor transplantation on the right side, all animals were euthanized to end the experiment. After completion of the experiment, the presence or absence of transplanted tumor tissue was observed in the left flank of all subjects. The tumor was excised from the left flank, and immune cells in the tumor tissue were analyzed by using a flow cytometer (FACSVerse, BD). For the analysis of immune cells in the tumor tissue, the tumor tissue was separated into single cells by using gentle MACS C tubes (Cat No: 130-093-237, Miltenyi Biotec), and then stained with a LIVE/DEAD Fixable Aqua Dead Cell Stain (Cat No: L34957, Invitrogen), an Fc block (Cat No: 553142, BD), and CD45-PE-Cy7 (Cat No: 25-0451-82, eBioscience), TCRbeta-APC-eFluor780 (Cat No: 47-5961-82, Invitrogen), CD4-pacific blue (Cat No: 100428, Biolegend), CD8a-PerCP-Cy55 (Cat No: 100734, Biolegend), CD11b-A647 (Cat No: 101218, Biolegend) antibodies to be analyzed by flow cytometry. Ratios of CD4+ and CD8a+ T cells and CD11b+ cells in the tumor represent ratios of CD45+TCRbeta+CD4+, CD45+TCRbeta+CD8a+, and CD45+CD11b+ cells based on viable cells.

After the tumor transplantation, when the sum of the right and left tumor sizes of each mouse reached 2,000 mm$^3$, the corresponding subjects were recorded as dead.

A Graphpad Prism (version 7.05, GraphPad Software Inc., CA, USA) was used for the graph presentation, and an SPSS software (version 25.0, SPSS Inc, IL, USA) and an Excel (2013, MS, USA) were used for the statistical analysis. A two-tailed t-test was performed with respect to the tumor size, a Pearson's Chi-Square test was performed with respect to the formation of left tumor, and Mantel-Cox log-rank analysis was performed with respect to the survival rates at a significance level of $p=0.05$.

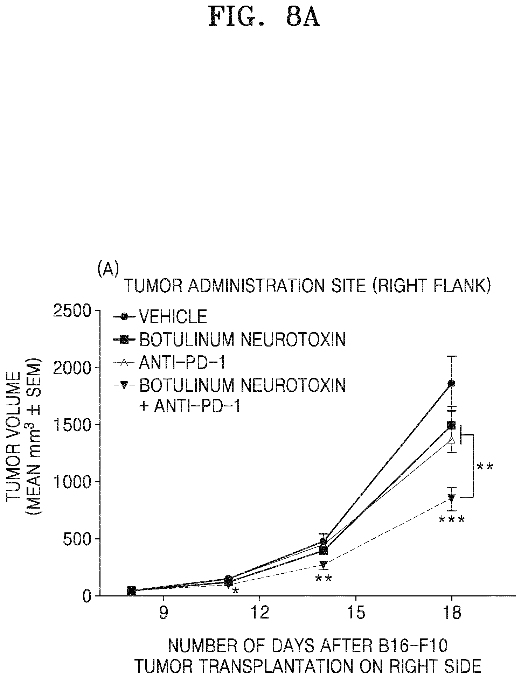

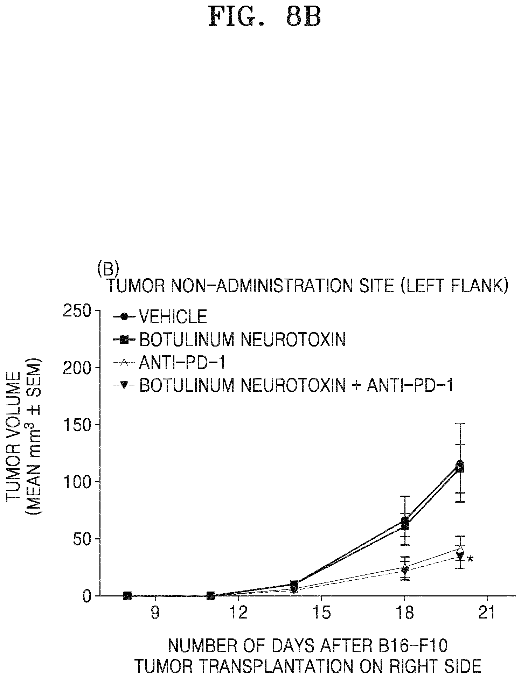

The tumor growth on the right and left sides over time was shown in terms of tumor volume (mm$^3$) as shown in FIGS. 8A and 8B (*$p<0.05$, **$p<0.01$, and ***$p<0.001$). When botulinum neurotoxin was intratumorally administered on the right side once at a dose of 15 U/kg and when 15 U/kg of botulinum neurotoxin was administered intraperitoneally in combination with 5 mg/kg of anti-PD-1 antibody, a significant reduction in the tumor size on the right side was observed in the mouse model transplanted with malignant melanoma on the right and left sides, compared to the test groups each administered with botulinum neurotoxin only and anti-PD-1 antibody only (FIG. 8A). Regarding the tumor size on the left side, the tumor size was significantly reduced in the test group administered with botulinum neurotoxin in combination with anti-PD-1 antibody, compared to the vehicle-administered group (FIG. 8B).

Table 7 shows the presence or absence of tumor formation on the left side (*$p<0.05$ vs G3 anti-PD-1 antibody).

[Table 7]

| Group | Material to be administered | Formation of tumor on left flank | |
|---|---|---|---|
| | | Observed (number of animals) | Not observed (number of animals) |
| 1 | Vehicle | 18 | 4 |
| 2 | 15 U/kg botulinum neurotoxin | 21 | 3 |
| 3 | Vehicle+5 mg/kg anti-PD-1 antibody | 18 | 2 |
| 4 | 15 U/kg botulinum neurotoxin+5 mg/kg anti-PD-1 antibody | 14 | 8* |

As shown in Table 7, a formation rate of the tumor on the left side was significantly reduced in the group administered with botulinum neurotoxin in combination with anti-PD-1 antibody, compared to the group administered with the anti-PD-1 antibody only.

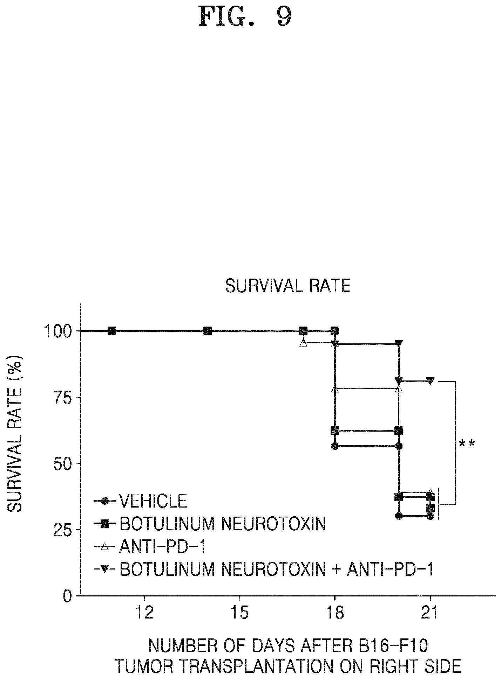

FIG. 9 shows the survival rate of the mice (**$p<0.01$), and it was confirmed that the survival rate was significantly improved in the test group administered with botulinum neurotoxin in combination with anti-PD-1 antibody.

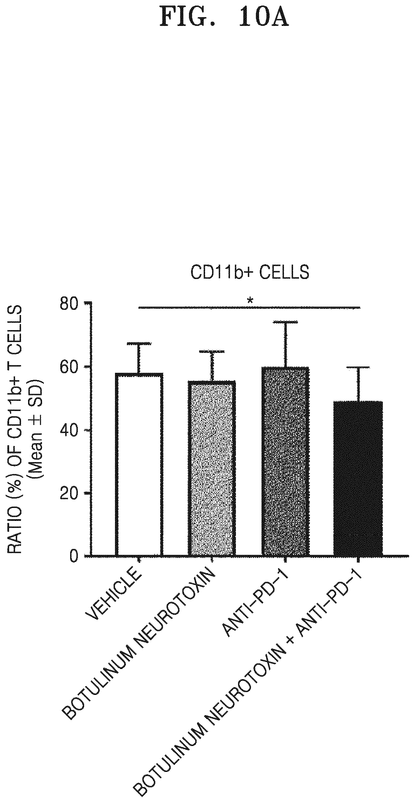

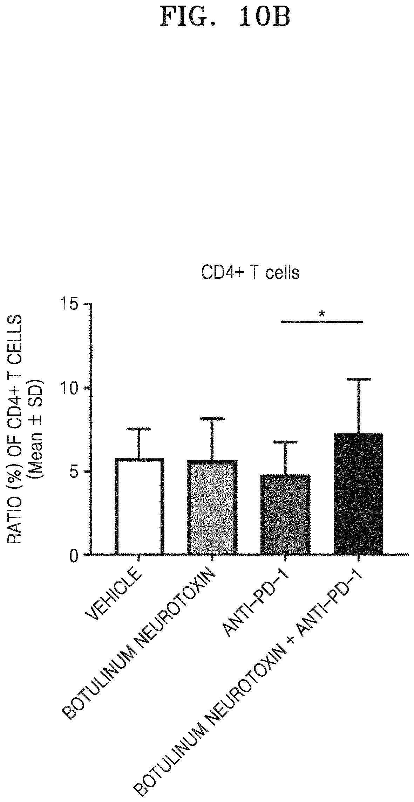

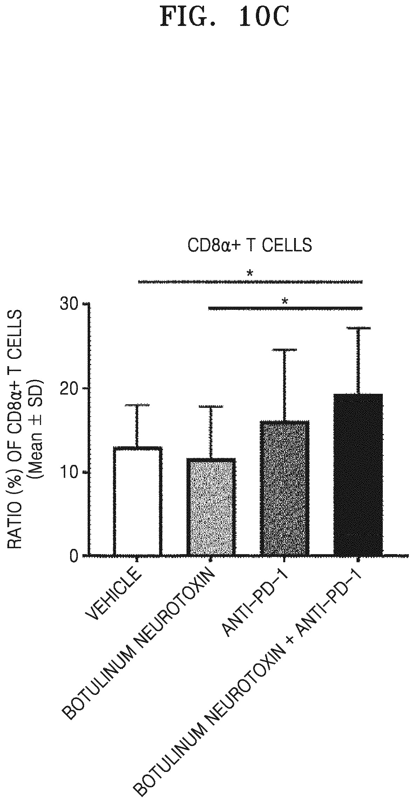

The ratio of CD11b+ cells and the ratio of CD4+ and CD8a+ T cells were calculated by using a two-tailed t-test (*$p$<0.05) based on viable cells infiltrated in the tumor on the left non-administered side. As a result of excising the tumor tissue transplanted on the left side and analyzing the CD4+ and CD8a+ T cells and CD11b+ cells infiltrated in the tumor, in the group administered with botulinum neurotoxin in combination with anti-PD1 antibody, a significant decrease in the CD11b+ cell ratio was observed compared to the vehicle-administered group (FIG. 10A), the CD4+ T cell ratio was significantly increased compared to the group administered with anti-PD-1 antibody only (FIG. 10B), and the CD8a+ T cell ratio was significantly increased compared to the vehicle-administered group and the group administered botulinum neurotoxin only (FIG. 10C).

Based on the results above, the significant improvement in the survival rate was confirmed when the mouse model of bilateral melanoma transplantation was administered with botulinum neurotoxin in combination with anti-PD-1 antibody. It was also confirmed that there were effects on not only the growth of tumor to which botulinum neurotoxin was administered, but also the formation and growth of tumor to which botulinum neurotoxin was not administered were confirmed.

**Example 6: Anti-cancer efficacy test of botulinum neurotoxin administered in combination with immuno-anticancer agent (anti-PD-1 and anti-CTLA4) on mouse model of colorectal cancer transplantation**

6-week-old female C57BL/6 mice were purchased (Orient Bio), and after acclimation and quarantine for 1 week, 7-week-old mice were used in experiments. For a feed, a sterilized solid feed for laboratory animals (R40-10, SAFE, France) was freely fed, and for drinking water, tap water was autoclaved and freely fed. During the periods of acclimatization, quarantine, and experiments, the mice were bred under specific-pathogen-free conditions set at a temperature of 23 ± 3 °C, relative humidity of 55 ± 15 %, lighting time of 12 hours (from 8:00 am to 8:00 pm), ventilation frequency of 15 times/hour, and illumination of 150 Lux to 300 Lux. This experiment was performed after review and approval (A-2020-004) by Institutional Animal Care and Use Committee of Medytox Inc.

A mouse colorectal cancer MC38 cell line (ENH204-FP) was obtained from Kerafast. The MC38 cell line was cultured in a DMEM medium (CAT No: 11965-092, Gibco) supplemented with 10 % FBS (CAT No: 10082-147, Gibco), 1 % penicillin-streptomycin (CAT No: 15140-122, Gibco), 10 mM HEPES (CAT No: 15630-080, Gibco), 0.1 mM MEM NEAA (CAT No: 11140-050, Gibco), and 1 mM sodium pyruvate (CAT No.: 11360-070, Gibco) in an incubator at 37 °C and 5 % $CO_2$ conditions. 0.1 mL of $2.5 \times 10^5$ cells were transplanted into the right flank of anesthetized C57BL/6 mice by using a syringe.

A placebo of a botulinum neurotoxin product containing the same excipient components as in the botulinum neurotoxin product, but only botulinum neurotoxin was excluded was used as a vehicle, and Coretox 100 unit (Lot No: NSA20015, Medytox) was used as botulinum neurotoxin. An anti-PD-1 antibody (Clone: RMP1-14, Cat No: BE0146, Lot No: 798921J1C) and an anti-CTLA4 antibody (Clone: 9H10, Cat No: BE0131, Lot No: 755620A2) were purchased from Bio X cell for use. Each test material was prepared and administered according to Table 6 below. Sterile physiological saline was added to a vial containing 100 units of botulinum neurotoxin to be prepared at a concentration of 15 units/mL, and then intratumorally administered at a dose of 1 mL/kg or 15 units/kg. Anti-PD-1 and anti-CTLA4 antibodies were intraperitoneally administered at a dose of 5 mg/kg with PBS (Cat No: LB004-02, Welgene). Propranolol (Cat No: P0884, Sigma) was prepared at a concentration of 2 mg/mL using PBS, and then intraperitoneally administered at a dose of 10 mg/kg.

On the 7th day after the mice were transplanted with colorectal cancer cell line, the tumor size was measured, and 10 mice were randomly assigned per test group to have no difference among the test groups. On the 7th day after the tumor transplantation, a vehicle and botulinum neurotoxin were intratumorally administered according to the compositions of the test groups. In the case of the anti-PD-1 and anti-CTLA4 antibodies, intraperitoneal administration thereof was performed twice a week for a total of 4 times according to Table 8. In the case of propranolol, intraperitoneal administration thereof was performed for a total of 8 times between the 7th day and the 16th day after the tumor transplantation, and the tumor size was measured twice a week after the tumor transplantation. The tumor size was measured with a vernier caliper, and the tumor volume was calculated by using the equation described in Example 1.

[Table 8]

| Group | Number of animal | Material to be administered | Dose | Administration route | Administration frequency |
|---|---|---|---|---|---|
| 1 | 10 | Vehicle | 1 ml/kg | Intratumoral | Once (on 7th day after tumor transplantation) |

(continued)

| Group | Number of animal | Material to be administered | Dose | Administration route | Administration frequency |
|---|---|---|---|---|---|
| 2 | 10 | 15 U/kg botulinum neurotoxin | 1 ml/kg | Intratumoral | Once (on 7th day after tumor transplantation) |
| 3 | 10 | Vehicle+5 mg/kg anti-PD-1 antibody | 1 ml/kg, 5 ml/kg | Intratumoral, intraperitoneal | Once (on 7th day after tumor transplantation), twice a week, 4 times in total |
| 4 | 10 | Vehicle+5 mg/kg anti-CTLA4 antibody | 1 ml/kg, 5 ml/kg | Intratumoral, intraperitoneal | Once (on 7th day after tumor transplantation), twice a week, 4 times in total |
| 5 | 10 | 15 U/kg botulinum neurotoxin+5 mg/kg anti-PD-1 antibody | 1 ml/kg, 5 ml/kg | Intratumoral, intraperitoneal | Once (on 7th day after tumor transplantation), twice a week, 4 times in total |
| 6 | 10 | 15 U/kg botulinum neurotoxin+5 mg/kg anti-CTLA4 antibody | 1 ml/kg, 5 ml/kg | Intratumoral, intraperitoneal | Once (on 7th day after tumor transplantation), twice a week, 4 times in total |
| 7 | 10 | Vehicle+10 mg/kg propranolol | 1 ml/kg, 5 ml/kg | Intratumoral, intraperitoneal | Once (on 7th day after tumor transplantation), 8 times in total (between 7th day to 16th day after tumor transplantation) |
| 8 | 10 | 10 mg/kg propranolol+5 mg/kg anti-PD-1 antibody | 10 ml/kg, 5 ml/kg | Intratumoral, intraperitoneal | 8 times in total (between 7th day to 16th day after tumor transplantation), twice a week, 4 times in total |

After the tumor transplantation, measurements were performed until the tumor size in each mouse reached 2,000 mm$^3$, and subjects having the tumor size exceeding the euthanasia standard (2,000 mm$^3$) were immediately euthanized and recorded as dead.

A Graphpad Prism (version 7.05, GraphPad Software Inc., CA, USA) was used for the graph presentation, and an SPSS software (version 25.0, SPSS Inc., IL, USA) and an Excel (2013, MS, USA) were used for the statistical analysis. A two-tailed t-test was performed with respect to the tumor size, and Mantel-Cox log-rank analysis was performed with respect to the survival rates at a significance level of p=0.05.

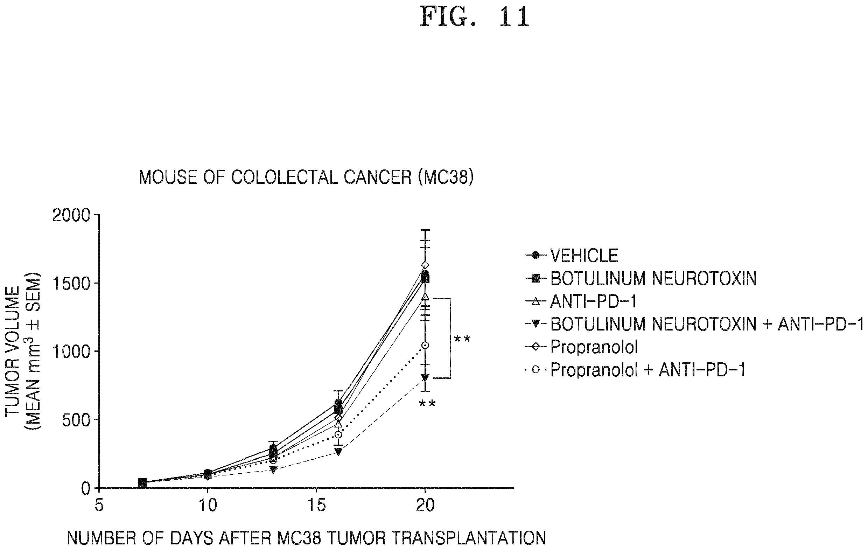

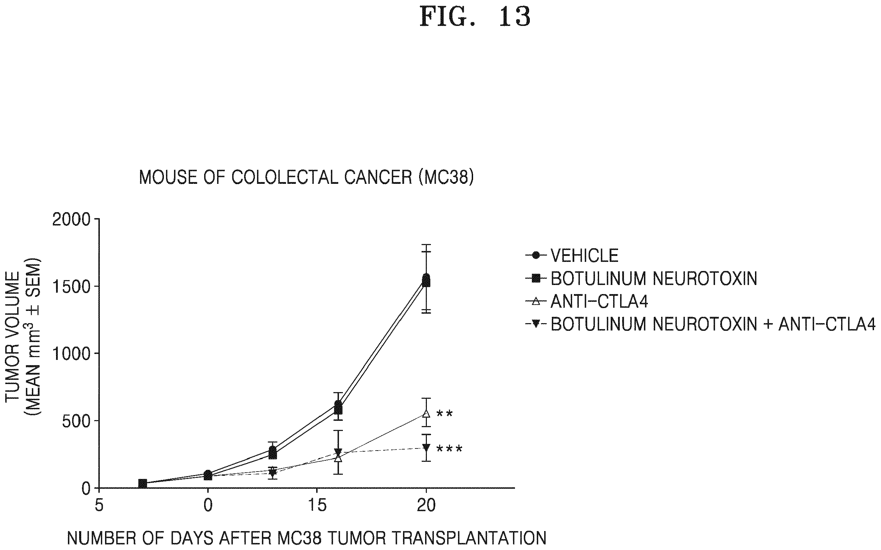

FIGS. 11 and 13 show the tumor growth over time represented in tumor volume (mm$^3$) (FIG. 11: **p<0.01 vs vehicle, and FIG. 13: **p<0.01 and ***p<0.001 vs vehicle). On the 20th day after the colorectal cancer transplantation on the mice, significant inhibition on the tumor growth was observed in the group administered with 15 U/kg of botulinum neurotoxin in combination with 5 mg/kg of anti-PD-1 antibody, compared to the vehicle-administered group, the group administered with botulinum neurotoxin only, and the group administered with anti-PD-1 only (FIG. 11). In addition, on the 20th day after the colorectal cancer transplantation, significant inhibition on the tumor growth was observed in the group administered with 5 mg/kg of anti-CTLA4 only and the group administered with botulinum neurotoxin in combination with anti-CTLA4, compared to the vehicle-administered group (FIG. 13).

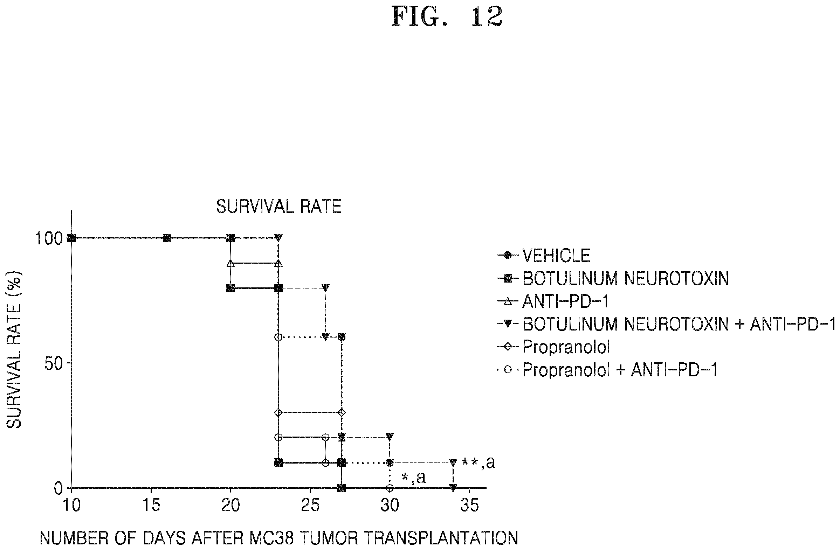

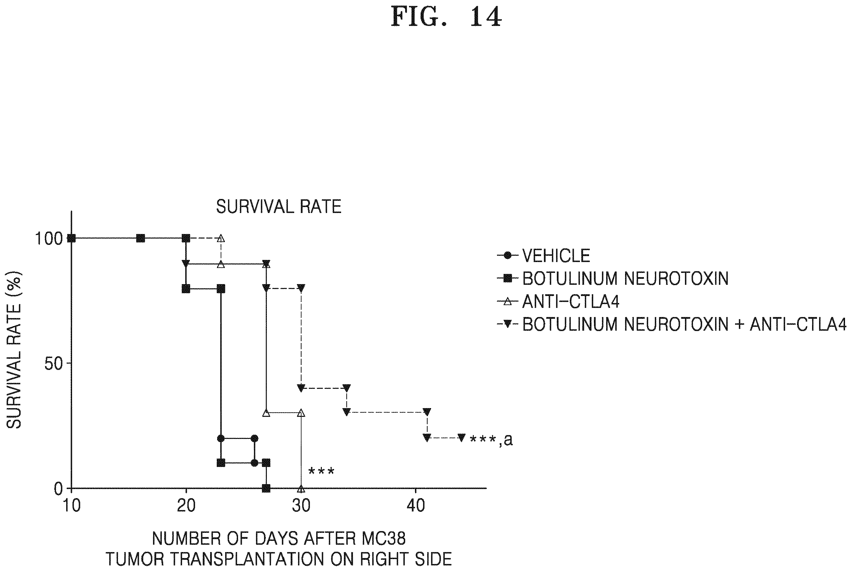

FIGS. 12 and 14 show the survival rates of mice (FIG. 12: *$p$<0.05, **$p$<0.01 vs vehicle, and $^a p$<0.05 vs anti-PD-1, and FIG. 14: ***$p$<0.001 vs vehicle and $^a p$<0.05 vs anti-CTLA4). In the test group administered with botulinum neurotoxin in combination with the anti-PD1 antibody, the survival rate of mice was significantly increased compared to the vehicle-administered group and the group administered with the anti-PD-1 antibody only. Also, in the test group administered with 10 mg/kg of propranolol in combination with the anti-PD1 antibody, the survival rate of mice was significantly increased compared to the vehicle-administered group and the group administered with the anti-PD-1 antibody only. A significant increase in the survival rate of mice was observed also in the group administered with the anti-CTLA4 antibody only. Also, a significantly increased survival rate of the mice was observed in the group administered with botulinum neurotoxin in combination with the anti-CTLA4 antibody, compared to the vehicle-administered group and the group administered with the anti-CTLA4 antibody only (FIG. 14).

Based on the results above, the synergistic effect on the tumor growth inhibition and the survival rate improvement were observed in the mouse model of colorectal cancer due to the administration of botulinum neurotoxin in combination with the anti-PD-1 or anti-CTLA4 immuno-anticancer agent. In addition, the synergistic effect by the administration of a beta-blocker (propranolol) in combination with an immuno-anticancer agent was observed, and when botulinum neurotoxin was administered in combination with an immuno-anticancer agent, the equivalent or better effects were confirmed compared to the effects of the group administered with the beta-blocker in combination with the immuno-anticancer agent.

**Claims**

1. A composition for cancer treatment comprising botulinum neurotoxin as an active ingredient and used in combination with an immunotherapeutic agent.

2. The composition of claim 1, wherein the botulinum neurotoxin comprises serotype A, B, C, D, E, F, G, H, or a combination thereof.

3. The composition of claim 1 or 2, wherein the immunotherapeutic agent is an immune checkpoint inhibitor.

4. The composition of claim 3, wherein the immune checkpoint inhibitor is a PD-1 antagonist, a PD-L1 antagonist, a CTLA-4 antagonist, a TIM3 antagonist, an LAG3 antagonist, a TIGIT antagonist, a VISTA antagonist, a BTLA antagonist, or a combination thereof.

5. The composition of claim 4, wherein the immune checkpoint inhibitor is at least one selected from the group consisting of an anti-PD-1 antibody, an anti-PD-L1 antibody, an anti-CTLA4 antibody, an anti-TIM3 antibody, an anti-LAG3 antibody, an anti-TIGIT antibody, an anti-VISTA antibody, an anti-BTLA antibody, or an antigen-binding fragment thereof.

6. The composition of claim 5, wherein the immune checkpoint inhibitor is an anti-PD-1 antibody, an anti-PD-L1 antibody, an anti-CTLA4 antibody, or an antigen-binding fragment thereof.

7. The composition of any one of claims 1 to 6, wherein cancer is exosome-mediated.

8. The composition of any one of claims 1 to 7, wherein cancer is a solid tumor.

9. The composition of claim 8, wherein the solid tumor is at least one selected from the group consisting of melanoma, colorectal cancer, breast cancer, lung cancer, pancreatic cancer, prostate cancer, bladder cancer, and stomach cancer.

10. The composition of any one of claims 1 to 9, wherein cancer is metastatic cancer.

11. The composition of any one of claims 1 to 10, wherein the composition is used in a subject having increased secretion of exosomes.

12. The composition of any one of claims 1 to 11, further comprising a pharmaceutically acceptable excipient or additive.

13. The composition of claim 12, wherein the pharmaceutically acceptable excipient or additive is a stabilizer, an ionic compound, a surfactant, a buffer agent, a lyoprotectant, or a combination thereof.

14. The combination of claim 13, wherein the pharmaceutically acceptable excipient or additive is an amino acid, a salt, a buffer solution, a non-ionic surfactant, a sugar, a sugar alcohol, or a combination thereof.

15. The composition of any one of claims 12 to 14, wherein albumin or an animal-derived ingredient or polysaccharide is not comprised.

16. The composition of any one of claims 12 to 15, wherein the composition is in the form of a lyophilized powder, a liquid, or a pre-filled syringe formulation.

17. The composition of any one of claims 12 to 16, wherein the composition is for local administration.

18. The composition of claim 17, wherein the composition is for intratumoral or peritumoral administration.

19. The composition of any one of claims 1 to 18, wherein the composition comprises the botulinum neurotoxin in a range of about 0.01 units/kg to about 100 units/kg.

20. The composition of any one of claims 1 to 19, wherein the botulinum neurotoxin is administered simultaneously or sequentially with the immunotherapeutic agent.

21. The composition of any one of claims 1 to 20, wherein the botulinum neurotoxin is administered with the immunotherapeutic agent in the form of a single formulation or separate formulations.

22. The composition of any one of claims 1 to 21, wherein the immunotherapeutic agent is administered parenterally.

23. The composition of claim 22, wherein the immunotherapeutic agent is locally administered or injected intravenously, intraperitoneally, subcutaneously, intradermally, intramuscularly, or into the spine, spinal cavity, or rectum.

# FIG. 1

MOUSE OF MALIGNANT MELANOMA (B16-F10)

# FIG. 2A

FIG. 2B

FIG. 2C

CD4+ T CELLS

# FIG. 2D

CD8+ T CELLS

# FIG. 3

FIG. 4

EP 4 186 520 A1

MOUSE OF MALIGNANT MELANOMA (B16−F10)

G1: VEHICLE
G2: 1 U/kg BOTULINUM NEUROTOXIN
G3: 3 U/kg BOTULINUM NEUROTOXIN
G4: 10 U/kg BOTULINUM NEUROTOXIN
G5: 1 U/kg BOTULINUM NEUROTOXIN
+ 5 mg/kg ANTI−PD−1
G6: 3 U/kg BOTULINUM NEUROTOXIN
+ 5 mg/kg ANTI−PD−1
G7: 10 U/kg BOTULINUM NEUROTOXIN
+ 5 mg/kg ANTI−PD−1
G8: VEHICLE + 5 mg/kg ANTI−PD−1

TUMOR VOLUME (MEAN mm$^3$ ± SEM)

NUMBER OF DAYS AFTER B16−F10 TUMOR TRANSPLANTATION

## FIG. 5

SURVIVAL RATE

NUMBER OF DAYS AFTER B16-F10 TUMOR TRANSPLANTATION

- G1: VEHICLE
- G2: 1 U/kg BOTULINUM NEUROTOXIN
- G3: 3 U/kg BOTULINUM NEUROTOXIN
- G4: 10 U/kg BOTULINUM NEUROTOXIN
- G5: 1 U/kg BOTULINUM NEUROTOXIN + 5 mg/kg ANTI-PD-1
- G6: 3 U/kg BOTULINUM NEUROTOXIN + 5 mg/kg ANTI-PD-1
- G7: 10 U/kg BOTULINUM NEUROTOXIN + 5 mg/kg ANTI-PD-1
- G8: VEHICLE + 5 mg/kg ANTI-PD-1

# FIG. 6

Y-axis: TUMOR VOLUME (MEAN mm³ ± SEM), marked at 0, 500, 1000, 1500, 2000, 2500

X-axis: NUMBER OF DAYS AFTER B16-F10 TUMOR TRANSPLANTATION, marked at 9, 12, 15, 18

Legend:
- G1: VEHICLE
- G2: 15 U/kg NON-COMPLEXED BOTULINUM NEUROTOXIN
- G3: 15 U/kg COMPLEXED BOTULINUM NEUROTOXIN
- G4: 15 U/kg NON-COMPLEXED BOTULINUM NEUROTOXIN + 5 mg/kg ANTI-PD-1
- G5: 15 U/kg COMPLEXED BOTULINUM NEUROTOXIN + 5 mg/kg ANTI-PD-1
- G6: VEHICLE + 5 mg/kg ANTI-PD-1

Data labels: *G4, *G5, *G4, **G2,5, **G2,4

# FIG. 7

# FIG. 8A

(A) TUMOR ADMINISTRATION SITE (RIGHT FLANK)

NUMBER OF DAYS AFTER B16-F10
TUMOR TRANSPLANTATION ON RIGHT SIDE

# FIG. 8B

(B)
TUMOR NON-ADMINISTRATION SITE (LEFT FLANK)

Legend:
- ●– VEHICLE
- ■– BOTULINUM NEUROTOXIN
- △– ANTI-PD-1
- ▼– BOTULINUM NEUROTOXIN + ANTI-PD-1

Y-axis: TUMOR VOLUME (MEAN $mm^3$ ± SEM)

X-axis: NUMBER OF DAYS AFTER B16-F10 TUMOR TRANSPLANTATION ON RIGHT SIDE

# FIG. 9

SURVIVAL RATE

SURVIVAL RATE (%)

VEHICLE
BOTULINUM NEUROTOXIN
ANTI-PD-1
BOTULINUM NEUROTOXIN + ANTI-PD-1

NUMBER OF DAYS AFTER B16-F10
TUMOR TRANSPLANTATION ON RIGHT SIDE

# FIG. 10A

CD11b+ CELLS

# FIG. 10B

CD4+ T cells

# FIG. 10C

CD8α+ T CELLS

FIG. 11

FIG. 12

SURVIVAL RATE

EP 4 186 520 A1

# FIG. 13

MOUSE OF COLOLECTAL CANCER (MC38)

Graph legend:
- VEHICLE
- BOTULINUM NEUROTOXIN
- ANTI-CTLA4
- BOTULINUM NEUROTOXIN + ANTI-CTLA4

Y-axis: TUMOR VOLUME (MEAN mm³ ± SEM), 0 to 2000

X-axis: NUMBER OF DAYS AFTER MC38 TUMOR TRANSPLANTATION, 5, 0, 15, 20

** , ***

FIG. 14

SURVIVAL RATE

SURVIVAL RATE (%)

- ● VEHICLE
- ■ BOTULINUM NEUROTOXIN
- △ ANTI-CTLA4
- ▼ BOTULINUM NEUROTOXIN + ANTI-CTLA4

***,a

***

NUMBER OF DAYS AFTER MC38
TUMOR TRANSPLANTATION ON RIGHT SIDE

## INTERNATIONAL SEARCH REPORT

International application No.

**PCT/KR2021/009225**

**A.    CLASSIFICATION OF SUBJECT MATTER**

**A61K 38/48**(2006.01)i; **A61K 39/395**(2006.01)i; **A61K 45/06**(2006.01)i; **A61P 35/00**(2006.01)i; **A61P 35/04**(2006.01)i; **C07K 16/28**(2006.01)i; **A61K 39/00**(2006.01)i

According to International Patent Classification (IPC) or to both national classification and IPC

**B.    FIELDS SEARCHED**

Minimum documentation searched (classification system followed by classification symbols)

A61K 38/48(2006.01); A61K 38/16(2006.01); A61K 39/08(2006.01); A61P 35/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 보툴리눔 독소(botulinum neurotoxin), 면역요법제(immunotherapeutic agent), 면역관문 억제제(immune checkpoint inhibitor), 암(cancer)

**C.    DOCUMENTS CONSIDERED TO BE RELEVANT**

| Category* | Citation of document, with indication, where appropriate, of the relevant passages | Relevant to claim No. |
|---|---|---|
| X | WO 2018-022668 A2 (FLAGSHIP PIONEERING, INC.) 01 February 2018 (2018-02-01)<br>See claims 1, 77-80 and 105-107; page 12, lines 30-37; page 19, lines 1-8; page 239, lines 14-20; and table 9. | 1-6 |
| X | US 2010-0172939 A1 (SHAARI, C.) 08 July 2010 (2010-07-08)<br>See claims 1-4 and 28. | 1,2 |
| A | KR 10-2007-0045292 A (ALLERGAN, INC.) 02 May 2007 (2007-05-02)<br>See entire document. | 1-6 |
| A | KOPENHAVER, J. et al. Mobilizing toxins for cancer treatment: historical perspectives and current strategies. Toxins. 23 June 2020, vol. 12, no. 416, pp. 1-5.<br>See entire document. | 1-6 |
| A | SPAIN, L. et al. The neurotoxic effects of immune checkpoint inhibitor therapy for melanoma. Melanoma Management. 2019, vol. 6, no. 2, MMT16(ch. 1-4).<br>See entire document. | 1-6 |

☐ Further documents are listed in the continuation of Box C.    ☑ See patent family annex.

| | |
|---|---|
| *      Special categories of cited documents:<br>"A"    document defining the general state of the art which is not considered to be of particular relevance<br>"D"    document cited by the applicant in the international application<br>"E"    earlier application or patent but published on or after the international filing date<br>"L"    document which may throw doubts on priority claim(s) or which is cited to establish the publication date of another citation or other special reason (as specified)<br>"O"    document referring to an oral disclosure, use, exhibition or other means<br>"P"    document published prior to the international filing date but later than the priority date claimed | "T"    later document published after the international filing date or priority date and not in conflict with the application but cited to understand the principle or theory underlying the invention<br>"X"    document of particular relevance; the claimed invention cannot be considered novel or cannot be considered to involve an inventive step when the document is taken alone<br>"Y"    document of particular relevance; the claimed invention cannot be considered to involve an inventive step when the document is combined with one or more other such documents, such combination being obvious to a person skilled in the art<br>"&"    document member of the same patent family |

| Date of the actual completion of the international search | Date of mailing of the international search report |
|---|---|
| **28 October 2021** | **28 October 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

| International application No. |
| --- |
| **PCT/KR2021/009225** |

**Box No. II    Observations where certain claims were found unsearchable (Continuation of item 2 of first sheet)**

This international search report has not been established in respect of certain claims under Article 17(2)(a) for the following reasons:

1. ☐  Claims Nos.:
because they relate to subject matter not required to be searched by this Authority, namely:

2. ☑  Claims Nos.: **9,13,14,18,23**
because they relate to parts of the international application that do not comply with the prescribed requirements to such an extent that no meaningful international search can be carried out, specifically:

   Claims 9, 13, 14, 18 and 23 refer to multiple dependent claims not meeting the requirement of PCT Rule 6.4(a).

3. ☑  Claims Nos.: **7,8,10-12,15-17,19-22**
because they are dependent claims and are not drafted in accordance with the second and third sentences of Rule 6.4(a).

**INTERNATIONAL SEARCH REPORT**
Information on patent family members

International application No.

**PCT/KR2021/009225**

| Patent document cited in search report | | | Publication date (day/month/year) | Patent family member(s) | | | Publication date (day/month/year) |
|---|---|---|---|---|---|---|---|
| WO | 2018-022668 | A2 | 01 February 2018 | EP | 3490542 | A1 | 05 June 2019 |
| | | | | EP | 3490581 | A2 | 05 June 2019 |
| | | | | EP | 3506926 | A1 | 10 July 2019 |
| | | | | US | 2019-0240293 | A1 | 08 August 2019 |
| | | | | US | 2021-0154272 | A1 | 27 May 2021 |
| | | | | US | 2021-0177823 | A1 | 17 June 2021 |
| | | | | WO | 2018-022664 | A1 | 01 February 2018 |
| | | | | WO | 2018-022664 | A8 | 26 April 2018 |
| | | | | WO | 2018-022666 | A1 | 01 February 2018 |
| | | | | WO | 2018-022668 | A3 | 18 April 2019 |
| US | 2010-0172939 | A1 | 08 July 2010 | EP | 1802339 | A2 | 04 July 2007 |
| | | | | EP | 1802339 | B1 | 21 September 2011 |
| | | | | EP | 2168594 | A1 | 31 March 2010 |
| | | | | EP | 2168594 | B1 | 29 April 2015 |
| | | | | EP | 2298339 | A1 | 23 March 2011 |
| | | | | EP | 2298339 | B1 | 15 July 2015 |
| | | | | EP | 2370090 | A1 | 05 October 2011 |
| | | | | EP | 2370090 | B1 | 25 May 2016 |
| | | | | EP | 2985034 | A1 | 17 February 2016 |
| | | | | EP | 3124038 | A1 | 01 February 2017 |
| | | | | US | 2009-0028972 | A1 | 29 January 2009 |
| | | | | US | 2010-0209456 | A1 | 19 August 2010 |
| | | | | US | 2013-0177548 | A1 | 11 July 2013 |
| | | | | US | 2013-0236497 | A1 | 12 September 2013 |
| | | | | US | 7709440 | B2 | 04 May 2010 |
| | | | | US | 8343929 | B2 | 01 January 2013 |
| | | | | US | 8435951 | B2 | 07 May 2013 |
| | | | | WO | 2006-034404 | A2 | 30 March 2006 |
| | | | | WO | 2006-034404 | A3 | 10 August 2006 |
| | | | | WO | 2010-062955 | A1 | 03 June 2010 |
| | | | | WO | 2011-066316 | A2 | 03 June 2011 |
| | | | | WO | 2011-066316 | A9 | 15 September 2011 |
| KR | 10-2007-0045292 | A | 02 May 2007 | AU | 2003-225549 | A1 | 06 September 2004 |
| | | | | AU | 2003-225549 | B2 | 16 November 2006 |
| | | | | AU | 2005-280574 | A1 | 09 March 2006 |
| | | | | AU | 7085200 | A | 18 June 2001 |
| | | | | AU | 7600501 | A | 13 February 2002 |
| | | | | CA | 2478902 | A1 | 26 August 2004 |
| | | | | CA | 2478902 | C | 01 May 2012 |
| | | | | CA | 2580748 | A1 | 09 March 2006 |
| | | | | CN | 101031317 | A | 05 September 2007 |
| | | | | CN | 101031317 | B | 04 July 2012 |
| | | | | EP | 1492561 | A1 | 05 January 2005 |
| | | | | EP | 1492561 | B1 | 28 June 2006 |
| | | | | EP | 1728517 | A2 | 06 December 2006 |
| | | | | EP | 1728517 | A3 | 25 June 2008 |
| | | | | EP | 1804827 | A1 | 11 July 2007 |
| | | | | EP | 1804827 | B1 | 10 December 2008 |
| | | | | EP | 1990059 | A2 | 12 November 2008 |
| | | | | EP | 1990059 | A3 | 30 September 2009 |

Form PCT/ISA/210 (patent family annex) (July 2019)

**INTERNATIONAL SEARCH REPORT**
Information on patent family members

International application No.

**PCT/KR2021/009225**

| Patent document cited in search report | Publication date (day/month/year) | Patent family member(s) | | | Publication date (day/month/year) |
|---|---|---|---|---|---|
| | | EP | 2204183 | A1 | 07 July 2010 |
| | | ES | 2264041 | T3 | 16 December 2006 |
| | | ES | 2317284 | T3 | 16 April 2009 |
| | | JP | 2006-510723 | A | 30 March 2006 |
| | | JP | 2008-511627 | A | 17 April 2008 |
| | | JP | 2012-025775 | A | 09 February 2012 |
| | | JP | 4889220 | B2 | 07 March 2012 |
| | | JP | 4922167 | B2 | 25 April 2012 |
| | | US | 106139845 | A | 31 October 2000 |
| | | US | 2002-0094339 | A1 | 18 July 2002 |
| | | US | 2005-0031648 | A1 | 10 February 2005 |
| | | US | 2005-0260231 | A1 | 24 November 2005 |
| | | US | 6350455 | B1 | 26 February 2002 |
| | | US | 6368605 | B1 | 09 April 2002 |
| | | US | 7838007 | B2 | 23 November 2010 |
| | | US | 7838008 | B2 | 23 November 2010 |
| | | US | 7846456 | B2 | 07 December 2010 |
| | | WO | 01-41790 | A1 | 14 June 2001 |
| | | WO | 02-09743 | A1 | 07 February 2002 |
| | | WO | 2004-071525 | A1 | 26 August 2004 |
| | | WO | 2006-025976 | A1 | 09 March 2006 |

Form PCT/ISA/210 (patent family annex) (July 2019)

**REFERENCES CITED IN THE DESCRIPTION**

**Patent documents cited in the description**

- WO 2010062955 A
- WO 2006094539 A
- WO 2009008595 A1
- WO 2012134240 A2